# EUROPEAN PATENT APPLICATION

(11) **EP 4 177 345 A1**
(43) Date of publication of application: **10.05.2023**
(21) Application number: 21838580.5
(22) Date of filing: 06.07.2021
(51) Int. Cl.: C12N 15/113, C12N 15/10, C12N 15/11

(54) **IMPROVED RNA EDITING METHOD**

(30) Priority: 06.07.2020 WO PCT/CN2020/100467
(71) Applicant: EdiGene Therapeutics (Beijing) Inc., Beijing 102206 (CN)
(72) Inventor: YUAN, Pengfei, Beijing 102206 (CN); YI, Zexuan, Beijing 102206 (CN); LIU, Nengyin, Beijing 102206 (CN)
(74) Representative: Lavoix
(86) International application number: PCT/CN2021/104801
(87) International publication number: WO 2022/007803

(57) **Abstract**

Provided is a method for editing a target RNA at a target residue position of a host cell. The method comprises introducing ADAR-recruiting RNA (arRNA) or a construct encoding the arRNA into a host cell, wherein the arRNA comprises a complementary RNA sequence that hybridizes to a target RNA; the target residue is located in a three-base motif comprising a 5' nearest-neighbor residue (upstream residue) of the target residue in the target RNA, the target residue, and a 3' nearest-neighbor residue (downstream residue) of the target residue in the target RNA, wherein the three-base motif is not UAG, and the complementary RNA sequence comprises a mismatch directly opposite to the upstream residue or the downstream residue in the target RNA. Further provided are arRNA for the method, an RNA obtained by the method, a host cell comprising the RNA, and use of the method in the treatment of a disease.

## Description

### TECHNICAL FIELD

The present application belongs to the field of gene editing, and particularly to the field of RNA editing, and it comprises introducing deaminase-recruiting RNA (dRNA, also referred to as arRNA) or a construct encoding the arRNA into a host cell to edit target RNA at a target residue position in the host cell.

### BACKGROUND ART

### CRISPR Technology

In recent years, genome editing technologies, led by clustered regularly interspaced short palindromic repeats (CRISPR, WO2014018423A3), have been developing rapidly, and have had a profound impact on many fields of biology and medicine. Many scientific researchers and biotech companies are also working to apply this technology to the clinic. In September 2019, Professor Deng Hongkui of Peking University and his collaborators published an article that first reported clinical trial results of editing stem cells by CRISPR and infusing the edited stem cells back into patients to treat acquired immunodeficiency syndrome (Aids) and leukemia, making great contributions to the transformation of CRISPR to the direction of gene therapy.

Despite the great potential application prospects of CRISPR, it also has a series of defects that make its transformation from the scientific research stage to clinical therapeutic application difficult. One of the problems is a core acting enzyme used in CRISPR: Cas9. CRISPR-based DNA editing requires the introduction of exogenously expressed Cas9 or other nucleases with similar function, which results in the following problems. Firstly, the nuclease requiring to be exogenously expressed usually has relatively high molecular weight, which makes the efficiency of delivering it into the body via a virus vector decreasing dramatically. Secondly, the expression of Cas9 has been shown to be a potential cancer risk in multiple studies. p53 is the most extensively studied tumor suppressor gene, the study of Haapaniemi et al. has revealed that the Cas9 system can activate p53-induced DNA damage (Haapaniemi et al., 2018), and Enache et al. have also found that the overexpression of Cas9 protein can selectively enrich cells with p53 inactivation mutations (Enache et al., 2020). In addition, Adikusuma has found that Cas9-edited mouse zygotes have a large number of large DNA deletions (Adikusuma et al., 2018), and Cullot et al. have further found that the Cas9-edited genome will have large fragment deletions including millions of bases, and more importantly, these deleted fragments include 5 proto-oncogenes and 7 tumor suppressor genes (Cullot et al., 2019). Finally, the exogenously expressed Cas9 is usually derived from bacteria e.g., *Staphylococcus aureus* and *Streptococcus pyogenes,* but does not naturally occur in humans or mammals, making it possible to elicit the immune response in the body of a patient. The study of Charlesworth et al. has revealed that IgG antibodies for Cas9 exist in human serum (Charlesworth et al., 2019). On the one hand, the antibodies may neutralize the exogenously expressed nuclease to inactive the nuclease, and on the other hand, the nuclease may cause damage or even toxicity to a patient or hinder further intervention treatment.

### A-to-I editing at the RNA level

In order to avoid potential risks in DNA editing, scientists also conduct studies on RNA editing. The genetic information in DNA needs to be transcribed into RNA and further translated into a protein to exert normal physiological functions, which is called the central dogma of organisms. Compared with editing at the DNA level, editing at the RNA level not only avoids damage to the genome, but also can change final biological functions. Common RNA editing is adenosine deaminases acting on RNA (ADAR)-mediated adenosine (A)-to-inosine (I) (guanosine) editing. In 2007, the research group of Professor Zhang Feng of Massachusetts Institute of Technology reported an RNA editing technology called RNA editing for programmable A to I replacement (REPAIR), which realized A-to-I editing of target RNA through an exogenously expressed Cas13-ADAR fusion protein and single guide RNA (sgRNA) (Cox et al., 2017). In this method, Cas13 binds to sgRNA to exert the targeting function so as to guide the fusion protein to a position to be edited, and meanwhile, the deaminating domain of ADAR exerts the catalytic function to realize A-to-I editing. However, similar to CRISPR, this method also requires the expression of an exogenous protein. The problems caused by the expression of an exogenous protein cannot be solved.

In order to solve the above problems and better apply the nucleic acid editing technology to the medical field, it is urgent to develop a novel nucleic acid editing technology, especially a novel technology independent of expression of an exogenous protein. In July 2019, the research group of Professor Wei Wensheng from School of Life Sciences, Peking University published an article entitled "Programmable RNA Editing by Recruiting Endogenous ADAR Using Engineered RNAs" on Nature Biotechnology, which first reported a novel nucleic acid editing technology: leveraging endogenous ADAR for programmable editing of RNA (LEAPER) (Qu et al., 2019) (WO2020074001A1). Different from CRISPR (WO2014018423A3) and REPAIR (WO2019005884A1), this technology gets rid of the dependence on overexpression of an exogenous nuclease in principle, making it have more advantages in the transformation to the medical field. However, this technology can only realize adenosine (A)-to-inosine (I) editing, i.e., adenosine (A)-to-guanosine (G) editing (because inosine (I) will be recognized as guanosine (G) during protein translation), so its application is still limited. Similar to CRISPR, this technology also requires a fragment of RNA serving as a guide to recruit an endogenous nuclease to a position to be edited. The fragment of guide RNA is named as ADAR-recruiting RNA (arRNA).

In January 2019, the research group of Thorsten Stafforst also reported a nucleic acid editing technology similar to LEAPER, which was named as recruiting endogenous ADAR to specific transcripts for oligonucleotide-mediated RNA editing (RESTORE, WO2020001793A1). Similar to LEAPER, RESTORE can also get rid of the dependence on an exogenous protein. However, different from LEAPER, firstly RESTORE can realize efficient editing only in the presence of IFN-γ that is a key factor in determining the development and severity of autoimmunity (Pollard et al., 2013), which greatly affects the application of this technology in the medical field. Secondly, RESTORE also requires a fragment of guide RNA that must be a chemically synthesized oligonucleotide necessary to artificially introduce numerous chemical modifications into it to ensure the stability. Among these chemical modifications, some of the modifications may have potential toxicity or immunogenicity, and some may also lead to different conformations of the same base chain, such that RNA with the same sequence may have dozens of different conformation combinations. By contrast, LEAPER can not only realize editing through chemically synthesized RNA, but also deliver it via a vector (such as an adeno-associated virus (AAV) and a lentivirus) into a cell of a patient to function, and thus the delivery means are flexible.

### Upstream and downstream residues or sequences adjacent to the A-to-I editing position

In DNA editing, an edited position will be delivered to all daughter cells by replication. Although the efficiency of editing at the DNA level is relatively low, edited cells can also be enriched by screening daughter cells or other methods. Different from DNA editing, in RNA editing, the resulting edited RNA are not inherited. Therefore, on the one hand, off-target sites in RNA editing cannot be inherited to offspring, making editing at the RNA level safer than DNA editing, and on the other hand, the efficiency of RNA editing is more important. In RNA editing of A-to-I, the REPAIR (WO2019005884A1), RESTORE (WO2020001793A1), or LEAPER (WO2020074001A1) system requires ADAR serving as a key enzyme in catalytic reaction. In mammalian cells, there are three types of ADAR proteins: ADAR1 (two isotypes: p110 and p150), ADAR2, and ADAR3 (without the catalytic activity). A catalytic substrate of ADAR protein is double-stranded RNA, ADAR protein can remove the -NH2 group from the adenosine (A) nucleobase to convert A to inosine (I) which is recognized as guanosine (G) and paired with cytidine (C) in the subsequent physiological processes of cells, for example, in the reverse transcription and translation processes or in the replication process of virus RNA in cells. Due to specific properties of ADAR, some similar factors affect the efficiencies of REPAIR, RESTORE, and LEAPER editing systems for RNA editing. One of the factors is upstream and downstream residues and sequences adjacent to the position to be edited. What bases are respectively the 5'-upstream and 3'-downstream bases adjacent to adenosine (A) (target A) to be edited (i.e., a target residue herein) in mRNA will obviously affect the editing efficiency. For convenience of description, a motif formed by linking a 5'-upstream base (upstream residue) adjacent to a target residue, a target residue, and a 3'-downstream base (downstream residue) adjacent to the target residue in the order of the 5'-end to the 3'-end is called a "three-base motif". Since the upstream residue or the downstream residue adjacent to the target A may be A, U, C or G, there are 16 three-base motifs: AAA, AAU, AAC, AAG, UAA, UAU, UAC, UAG, CAA, CAU, CAC, CAG, GAA, GAU, GAC, and GAG. Editing efficiencies of the REPAIR, RESTORE or LEAPER system for different three-base motifs are different, and this situation of different editing efficiencies for different three-base motifs is referred to as "three-consecutive-base preference" herein.

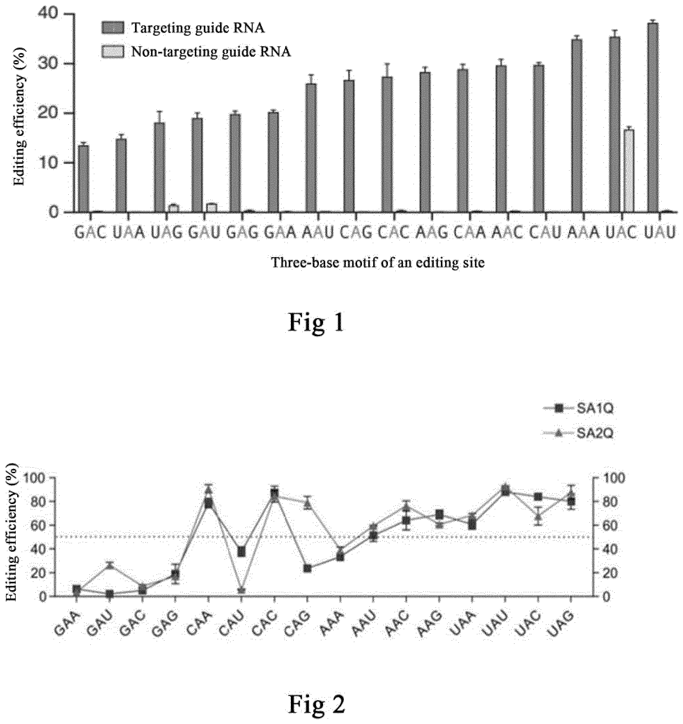

In the REPAIR system, since the Cas13-ADAR fusion protein is adopted, this system shows slightly different three-consecutive-base preference. As shown in Fig. 1 (Cox et al, 2017), the editing efficiency of the REPAIR system for the three-base motif of GAC is the lowest, and the editing efficiency for UAU is the highest, and the difference of editing efficiency is about 2-3 times.

In the RESTORE system, the authors of the article did not directly show data on preferences for three-base motifs, but cited another article (Vogel et al., 2018), and stated that the preference of the RESTORE system may be consistent with that of this system (Merkle et al., 2019). As shown in Fig. 2 (Vogel et al., 2018), the triangle in this figure represents SA1Q, the specific implementation method is as follows: a catalytic domain of human ADAR1 is fused with a C-terminal domain (SNAP-tag) of human O⁶-alkylguanine-DNA-alkyl transferase (hAGT), subjecting to mutation of glutamic acid to glutamine (E-Q) at the position 835 amino acid, and covalently cross-linking with guide RNA through SNAP-tag (Keppler, A. et al., 2003; Stafforst, T., et al., 2012); the square in this figure represents SA2Q, and the specific implementation method is as follows: a catalytic domain of human ADAR2 is fused with a C-terminal domain (SNAP-tag) of a human O⁶-alkylguanine-DNA-alkyl transferase (hAGT), subjecting to mutation of glutamic acid to glutamine (E-Q) at the position 1310 amino acid, and covalently cross-linking with guide RNA through SNAP-tag (Keppler, A. et al., 2003; Stafforst, T., et al., 2012). It can be seen that preferences of the two different types of ADAR show similar trends of obvious difference. In a case that the 5'-upstream residue is G, i.e., the editing efficiency for the three-base motif of GAA, GAU, GAC or GAG is usually far lower than the editing efficiencies for other three-base motifs and even close to the unedited level, and UAG is one of the three-base motifs with the highest editing efficiencies. As shown in Fig. 2, the editing efficiency of this system for UAG can be up to 10 times that of this system for the three-base motif having a upstream residue of G.

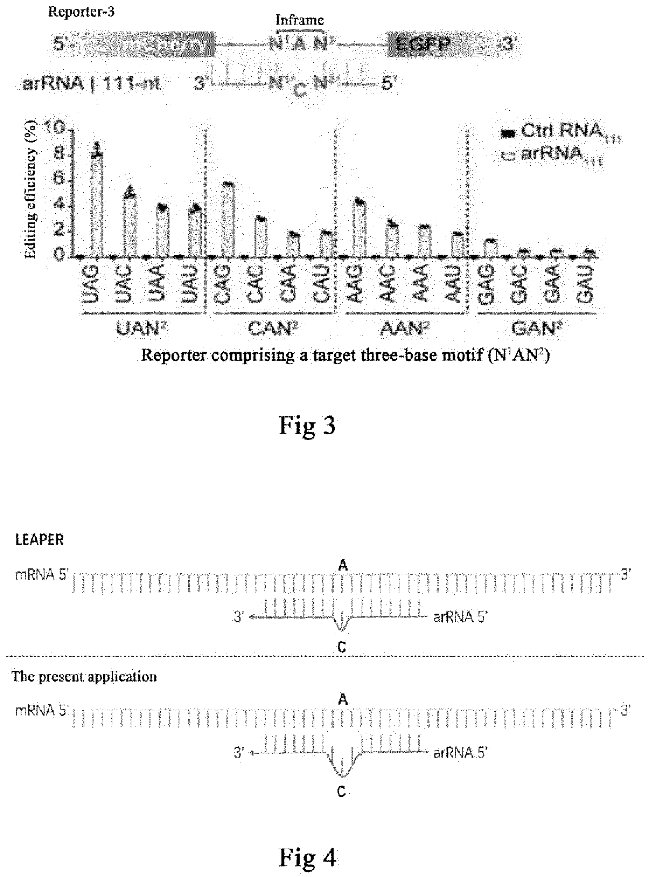

In the LEAPER system, the authors of the article directly tested the three-consecutive-base preference of this system (Qu et al., 2019). As shown in Fig. 3, in the LEAPER system, since it adopts the same complete and unmodified or unchanged ADAR as the RESTORE system, it is not difficult to understand that it shows similar three-consecutive-base preference to the RESTORE system. It can be seen from Fig. 3 that editing efficiencies of the LEAPER system for the three-base motifs of GAA, GAU, GAC, and GAG are also the lowest and even close to zero, the editing efficiency for the three-base motif of UAG is the highest, and similarly, the editing efficiency of the LEAPER system for UAG can also be up to more than 10 times that of the LEAPER system for the three-base motif having a upstream residue of G. In conclusion, in the REPAIR system, since the exogenously overexpressed Cas13-ADAR is adopted, the REPAIR system has slightly different three-consecutive-base preference. However, in the LEAPER system and the RESTORE system, since unmodified or unchanged ADAR is adopted, the two systems show similar three-consecutive-base preferences. Among all the three-base motifs, when unmodified or unchanged ADAR is used for editing, the editing efficiency for UAG is the highest, or UAG is one of the three-base motifs with the highest editing efficiencies. In a case that the 5'-upstream residue is G, the editing efficiency is obviously reduced and even close to zero, and the editing efficiency for UAG may be more than 10 times the editing efficiency for the three-base motif with a 5'-upstream residue of G. It indicates that the endogenous ADAR-based RNA editing systems in the prior art are almost unable to edit a position in a three-base motif having a 5'-upstream residue of G.

### SUMMARY OF THE APPLICATION

The three-consecutive-base preference of the technical systems performing RNA editing by deaminase in the prior art limits the application scope of the existing RNA editing technologies. For example, the existing RNA editing technologies are almost unable to edit a position in a three-base motif having a upstream residue of G, which greatly affects the application of these systems in the treatment of diseases. It is difficult to correct and treat an inherited disease caused by a pathogenic gene whose mutated position having an upstream residue of G by the known RNA editing means. However, the problem to be solved by the present application is, with respect to the three-base motifs other than the preferred three-base motif in the prior arts (e.g., the three-base motifs other than UAG), breaking through the limitation of the three-consecutive-base preference without modifying or changing the existing deaminase by adjusting the deaminase-recruiting RNA (dRNA or arRNA) sequence for recruiting a deaminase to target RNA to realize accurate editing, so as to greatly improve the editing efficiency for a three-base motif having an upstream residue of G or C.

Therefore, in one aspect, the present application provides a method for editing target RNA at a target residue position in a host cell, which comprises introducing ADAR-recruiting RNA (arRNA) or a construct encoding the arRNA into a host cell, wherein the arRNA comprises a complementary RNA sequence that hybridizes to a target RNA, and the target residue is located in a three-base motif comprising a 5' nearest-neighbor residue (upstream residue) of the target residue in the target RNA, the target residue, and a 3' nearest-neighbor residue (downstream residue) of the target residue in the target RNA, wherein the three-base motif is not UAG, and the complementary RNA sequence comprises a mismatch directly opposite to the upstream residue or the downstream residue in the target RNA.

In some embodiments, the present application provides a method for editing target RNA at a target residue position in a host cell, which comprises introducing ADAR-recruiting RNA (arRNA) or a construct encoding the arRNA into a host cell, wherein the arRNA comprises a complementary RNA sequence that hybridizes to a target RNA, and the target residue is located in a three-base motif comprises a 5' nearest-neighbor residue (upstream residue) of the target residue in the target RNA, the target residue, and a 3' nearest-neighbor residue (downstream residue) of the target residue in the target RNA; wherein the three-base motif is not UAG, and the complementary RNA sequence comprises mismatches directly opposite to the upstream residue and the downstream residue in the target RNA.

In certain embodiments, the upstream residue in the three-base motif is G. In certain embodiments, the upstream residue in the three-base motif is A. In certain embodiments, the upstream residue in the three-base motif is C. In certain embodiments, the downstream residue in the three-base motif is C. In certain embodiments, the downstream residue in the three-base motif is U. In certain embodiments, the downstream residue in the three-base motif is A. In certain embodiments, the three-base motif is selected from the group consisting of: GAG, GAC, GAA, GAU, AAG, AAC, AAA, AAU, CAG, CAC, CAA, CAU, UAA, UAC, and UAU.

According to the method of the present application, in some embodiments, in a case that the upstream residue in the three-base motif is G, the base opposite to the upstream residue in the complementary RNA is G. In some embodiments, in a case that the upstream residue in the three-base motif is G, the base opposite to the upstream residue in the complementary RNA is A. In some embodiments, the three-base motif is GAU, and three consecutive complementary bases (directly opposite to the three-base motif) comprised in the complementary RNA sequence are ACG or ACA. In some embodiments, the three-base motif is GAU, and three consecutive complementary bases (directly opposite to the three-base motif) comprised in the complementary RNA sequence are ACG. In some embodiments, the three-base motif is GAA, and three consecutive complementary bases (directly opposite to the three-base motif) comprised in the complementary RNA sequence are UCA, CCG, CCC or UCC. In certain embodiments, the three-base motif is GAA, and three consecutive complementary bases (directly opposite to the three-base motif) comprised in the complementary RNA sequence are UCA. In some embodiments, the three-base motif is GAC, and three consecutive complementary bases (directly opposite to the three-base motif) comprised in the complementary RNA sequence are GCG or GCA. In certain embodiments, the three-base motif is GAC, and three consecutive complementary bases (directly opposite to the three-base motif) comprised in the complementary RNA sequence are GCG. In some embodiments, the three-base motif is GAG, and three consecutive complementary bases (directly opposite to the three-base motif) comprised in the complementary RNA sequence are CCG, CCA, CCC, UCC or UCG. In certain embodiments, the three-base motif is GAG, and three consecutive complementary bases (directly opposite to the three-base motif) comprised in the complementary RNA sequence are CCG.

In some embodiments, the complementary RNA sequence comprises cytidine (C), adenosine (A) or uridine (U) directly opposite to the target adenosine in the target RNA. In some specific embodiments, the complementary RNA sequence comprises C directly opposite to the target adenosine in the target RNA.

According to the method of the present application, in some embodiments, the complementary RNA sequence that hybridizes to the target RNA further comprises one or more mismatches respectively opposite to a non-target adenosine in the target RNA. In certain embodiments, the mismatch nucleoside opposite to one or more non-target adenosines is guanosine.

In some embodiments, the upstream residue in the three-base motif is G, and the base opposite to the upstream residue in the complementary RNA is G or A. In some embodiments, the downstream residue in the three-base motif is strictly complementary to an opposite base in the complementary RNA. In some embodiments, the upstream residue in the three-base motif is G, wherein the base opposite to the upstream residue in the complementary RNA is G or A, and the downstream residue in the three-base motif is strictly complementary to an opposite base in the complementary RNA. In some embodiments, the complementary RNA sequence comprises C directly opposite to the target adenosine in the target RNA, and the upstream residue in the three-base motif is G, wherein the base opposite to the upstream residue in the complementary RNA is G or A, and the downstream residue in the three-base motif is strictly complementary to an opposite base in the complementary RNA. In some embodiments, the complementary RNA sequence comprises C directly opposite to the target adenosine in the target RNA, and the upstream residue in the three-base motif is G, wherein the base opposite to the upstream residue in the complementary RNA is G, and the downstream residue in the three-base motif is strictly complementary to an opposite base in the complementary RNA.

In the above RNA editing method of the present application, the RNA editing efficiency is increased by at least 90% to 1100%, e.g., at least 100%, 200%, 300%, 400%, 500%, 600%, 700%, 800%, 900%, and 1000%, as compared to the prior art.

In some embodiments, the target adenosine (A) in the target RNA is deaminated through an adenosine deaminase (adenosine deaminase acting on RNA, ADAR). In certain embodiments, the adenosine deaminase is natural ADAR, or a homologous protein thereof. In certain embodiments, the adenosine deaminase is an adenosine deaminase functional variant that is modified but retains the adenosine deaminase activity, e.g., a variant that is obtained by modifying natural ADAR or a homologous protein thereof by one or more position mutations and retains the adenosine deaminase activity. In certain embodiments, the adenosine deaminase is a fusion protein comprising an ADAR catalytic domain, or ADAR homologous protein catalytic domain, or adenosine deaminase functional variant. In certain embodiments, the fusion protein comprising an ADAR protein catalytic domain is a fusion protein comprising Cas13 protein that loses catalytic activity after mutation and an ADAR functional domain, or ADAR homologous protein functional domain, or adenosine deaminase functional variant. In some embodiments, the deaminase with the adenosine deaminase activity is exogenously introduced into a host cell or expressed in the host cell by introducing a construct encoding the deaminase. In certain embodiments, the fusion protein comprising an ADAR protein catalytic domain is a fusion protein comprising λN peptide and an ADAR functional domain, or ADAR homologous protein catalytic domain, or adenosine deaminase functional variant. In certain embodiments, the fusion protein comprising an ADAR protein catalytic domain is SNAP-tag-labelled ADAR, or a SNAP-tag-labelled ADAR functional variant. In certain embodiments, the ADAR is ADAR1 and/or ADAR2. In some embodiments, ADAR is one or more ADARs selected from the group consisting of: hADAR1, hADAR2, mouse ADAR1, and mouse ADAR2.

In certain embodiments, the ADAR is expressed by a host cell. In certain embodiments, ADAR naturally or endogenously occurs in a host cell, for example, ADAR naturally or endogenously occurs in a eukaryocyte. In certain embodiments, the ADAR protein is exogenously introduced into a host cell. In certain embodiments, the ADAR or a construct encoding the ADAR is introduced into a host cell. In some embodiments, the construct is any one selected from (but is not limited to): a linear nucleic acid, a plasmid, a virus, etc. In the above method, the ADAR comprises the above natural ADAR and a homologous protein thereof, an adenosine deaminase functional variant that is modified but retains the adenosine deaminase activity (e.g., a variant that is obtained by modifying natural ADAR or a homologous protein thereof by one or more position mutations and retains the adenosine deaminase activity), and a fusion protein comprising an ADAR catalytic domain, or ADAR homologous protein catalytic domain, or adenosine deaminase functional variant. In some embodiments, the method does not comprise the step of introducing any protein into a host cell. In certain embodiments, the ADAR is ADAR1 and/or ADAR2. In some embodiments, ADAR is one or more ADAR selected from the group consisting of: hADAR1, hADAR2, mouse ADAR1, and mouse ADAR2.

In another aspect, the present application provides a method for editing target RNA at a target residue position in a host cell, wherein the target residue is cytidine, and arRNA recruits a deaminase acting on RNA and having cytidine deaminase activity (or referred to as a "cytidine deaminase", in the present application, the deaminase with the cytidine deaminase activity and the cytidine deaminase have the same meaning and are interchangeable), so as to deaminate the target cytidine in target RNA. In some embodiments, the deaminase with the cytidine deaminase activity, or a construct encoding the deaminase with the cytidine deaminase activity is introduced into a host cell. According to the method, the arRNA comprises a complementary RNA sequence that hybridizes to the target RNA, and the target residue is located in a three-base motif comprising a 5' nearest-neighbor residue (upstream residue) of the target residue in the target RNA, the target residue, and a 3' nearest-neighbor residue (downstream residue) of the target residue in the target RNA, wherein the target residue is cytidine (C), and the complementary RNA sequence comprises a mismatch directly opposite to the upstream residue and/or the downstream residue in the target RNA.

In some embodiments, the three-base motif in which the target cytidine is located is selected from any one selected from the group consisting of: GCG, GCC, GCA, GCU, ACG, ACC, ACA, ACU, CCG, CCC, CCA, CCU, UCA, UCC, UCU, and UCG. In some embodiments, the arRNA comprises an unpaired nucleotide at a position corresponding to the target residue in the target RNA to form a mismatch with the target residue. In some embodiments, the complementary RNA sequence in the arRNA that can hybridize to the target RNA comprises cytidine, adenosine or uridine directly opposite to the target cytidine in the target RNA. In certain embodiments, the complementary RNA sequence comprises uridine directly opposite to the target cytidine. In certain embodiments, the arRNA comprises one or more unpaired nucleotides at positions corresponding to non-target editing positions in the target RNA to form one or more mismatches with the non-target positions in the target RNA.

In some embodiments, the upstream residue in the three-base motif is G, and the base opposite to the upstream residue in the complementary RNA is G. In some embodiments, the downstream residue in the three-base motif is A, and the base opposite to the downstream residue in the complementary RNA is U or A. In some embodiments, the three-base motif is ACA, and the complementary RNA sequence comprises AUU or GUU opposite to the three-base motif. In some embodiments, the three-base motif is ACA, and the complementary RNA sequence comprises AUU opposite to the three-base motif. In some embodiments, the three-base motif is UCA, and the complementary RNA sequence comprises AUA, GUA or CUA opposite to the three-base motif. In some embodiments, the three-base motif is UCA, and the complementary RNA sequence comprises AUA opposite to the three-base motif. In some embodiments, the three-base motif is GCA, and the complementary RNA sequence comprises UUG or UCG opposite to the three-base motif. In some embodiments, the three-base motif is GCA, and the complementary RNA sequence comprises UUG opposite to the three-base motif. In some embodiments, the three-base motif is CCA, and the complementary RNA sequence comprises AUG opposite to the three-base motif.

In some embodiments, the deaminase with the cytidine deaminase activity is a deaminase with the C-to-U catalytic activity that is obtained by gene modification of ADAR protein or a fusion protein comprising an ADAR catalytic domain. In certain embodiments, the cytidine deaminase is modified ADAR2, and comprises one or more mutant ADAR2 catalytic domains selected from: E488Q/V351 G/S486A/T37 5S/S370C/P462A/N 597I/L332I/I398V /K350I/M383L/D619G/S582T/V 440I/S495N/K418E/S661T. In certain embodiments, the cytidine deaminase is a fusion protein comprising all of the following mutant ADAR2 catalytic domains: E488Q/V351 G/S486A/T37 5S/S370C/P462A/N 597I/L332I/I398V /K350I/M383L/D619G/S582T/V 440I/S495N/K418E/S661T. In some embodiments, the deaminase with the cytidine deaminase activity further comprises a targeting domain. In certain embodiments, the targeting domain comprises, but is not limited to, any one selected from the group consisting of: Cas13 protein that loses catalytic activity after mutation, λN peptide, and SNAP-tag. The deaminase with the cytidine deaminase activity comprises Cas13 protein that loses catalytic activity after mutation. In some embodiments, the fusion protein comprises Cas13 protein that loses catalytic activity after mutation and an ADAR2 catalytic domain with the cytidine deaminase activity. In some embodiments, the deaminase with the cytidine deaminase activity is exogenously introduced into a host cell or expressed in the host cell by introducing a construct encoding the deaminase.

In certain embodiments, the method comprises the step of introducing the cytidine deaminase or the fusion protein or a construct encoding the cytidine deaminase or the fusion protein into a cell comprising target RNA, wherein the construct encoding the cytidine deaminase or the fusion protein is any one selected from (but is not limited to): a linear nucleic acid, a plasmid, a vector, etc. In certain embodiments, the target residue in a three-base motif in the target RNA is cytidine, and the upstream residue in the three-base motif is a nucleotide selected from G, C, A, and U, and the preferred order is as follows: G>C>A≈U.

According to the above method of the present application, the arRNA is single-stranded RNA. In some embodiments, the complementary RNA sequence is completely single-stranded. In certain embodiments, the arRNA comprises one or more (e.g., 1, 2, 3 or more) double-stranded regions and/or one or more stem-loop regions. In certain embodiments, the arRNA is composed of the complementary RNA sequence only.

According to the method of the present application, in some embodiments, the arRNA has a length of about 20-260 nucleotides, for example, the arRNA has a length of 40-260, 45-250, 50-240, 60-230, 65-220, 70-220, 70-210, 70-200, 70-190, 70-180, 70-170, 70-160, 70-150, 70-140, 70-130, 70-120, 70-110, 70-100, 70-90, 70-80, 75-200, 80-190, 85-180, 90-170, 95-160, 100-200, 100-150, 100-175, 110-200, 110-175, 110-150 or 105-140 nucleotides. In some embodiments, the arRNA has a length of about 60-200 (e.g., any one of about 60-150, 65-140, 68-130, and 70-120) nucleotides. In some embodiments, the arRNA further comprises an ADAR-recruiting domain.

According to the method of the present application, in some embodiments, the arRNA comprises one or more chemical modifications. In some embodiments, the chemical modifications comprise methylation and/or phosphorothioation, e.g., 2'-O-methylation (2'-O-Me) and/or an internucleotide phosphorothioate bond. In certain embodiments, the first and last 3 or 5 nucleotides in the arRNA comprise 2'-O-Me modifications, and/or the linkages between the first and last 3, 4 or 5 nucleotides comprise phosphorothioate bond modifications. In certain embodiments, one or more or all uridines in the arRNA comprise 2'-O-Me modifications. In certain embodiments, a targeting nucleoside in the arRNA, and/or a nucleoside (e.g., one or two nucleosides directly adjacent to the 5'-end and/or the 3'-end of the targeting nucleoside) adjacent to the 5'-end and/or the 3'-end of the targeting nucleoside comprise 2'-O-Me modifications. In certain embodiments, a targeting nucleoside in the arRNA, and/or a nucleoside (e.g., one or two nucleosides directly adjacent to the 5'-end and/or the 3'-end of the targeting nucleoside) adjacent to the 5'-end and/or the 3'-end of the targeting nucleoside comprise 3'-phosphorothioate bond modifications. In certain embodiments, the arRNA does not comprise any chemical modification.

The present application further provides edited RNA produced by the target RNA editing method of the present application, or a host cell comprising the edited RNA.

The method for editing target RNA at a target residue position in a host cell of the present application can be applied to treat or prevent a disease or condition in an individual. Therefore, the present application further provides a method for treating or preventing a disease or condition in an individual, which comprises the following steps: editing target RNA associated with a disease or condition in an individual cell by any method for editing target RNA at a target residue position in a host cell according to the present application. In some embodiments, the disease or condition is an inherited gene disease, or a disease or condition associated with one or more acquired gene mutations (e.g., drug resistance).

The present application further provides RNA (arRNA) that can be applied to the method of the present application for recruiting a deaminase acting on RNA to deaminate a target residue in target RNA, which comprises a complementary RNA sequence that hybridizes to the target RNA, wherein the target residue is located in a three-base motif comprising a 5' nearest-neighbor residue (upstream residue) of the target residue in the target RNA, the target residue, and a 3' nearest-neighbor residue (downstream residue) of the target residue in the target RNA, wherein the three-base motif is not UAG, and the complementary RNA sequence comprises a mismatch directly opposite to the upstream residue and/or the downstream residue in the target RNA.

According to the arRNA of the present application, the arRNA comprises C directly opposite to target adenosine in the target RNA. In certain embodiments, the arRNA that hybridizes to the target RNA further comprises one or more mismatches respectively opposite to a non-target adenosine in the target RNA. In certain embodiments, the mismatch opposite to one or more non-target adenosines is guanosine. In certain embodiments, the three-base motif is GAU, and three consecutive complementary bases (directly opposite to the three-base motif) comprised in the arRNA are ACG or ACA. In certain embodiments, the three-base motif is GAU, and three consecutive complementary bases (directly opposite to the three-base motif) comprised in the arRNA are ACG. In certain embodiments, the three-base motif is GAA, and three consecutive complementary bases (directly opposite to the three-base motif) comprised in the arRNA are UCA, CCG, CCC or UCC. In certain embodiments, the three-base motif is GAA, and three consecutive complementary bases (directly opposite to the three-base motif) comprised in the arRNA are UCA. In certain embodiments, the three-base motif is GAC, and three consecutive complementary bases (directly opposite to the three-base motif) comprised in the arRNA are GCG or GCA. In certain embodiments, the three-base motif is GAC, and three consecutive complementary bases (directly opposite to the three-base motif) comprised in the arRNA are GCG. In certain embodiments, the three-base motif is GAG, and three consecutive complementary bases (directly opposite to the three-base motif) comprised in the arRNA are CCG, CCA, CCC, UCC or UCG. In certain embodiments, the three-base motif is GAG, and three consecutive complementary bases (directly opposite to the three-base motif) comprised in the arRNA are CCG.

According to the arRNA of the present application, in some embodiments, the arRNA has a length of about 20-260 nucleotides, for example, the arRNA has a length of 40-260, 45-250, 50-240, 60-230, 65-220, 70-220, 70-210, 70-200, 70-190, 70-180, 70-170, 70-160, 70-150, 70-140, 70-130, 70-120, 70-110, 70-100, 70-90, 70-80, 75-200, 80-190, 85-180, 90-170, 95-160, 100-200, 100-150, 100-175, 110-200, 110-175, 110-150 or 105-140 nucleotides. In some embodiments, the arRNA has a length of about 60-200 (e.g., any one of about 60-150, 65-140, 68-130, and 70-120) nucleotides. In some embodiments, the arRNA further comprises an ADAR-recruiting domain.

According to the arRNA of the present application, in some embodiments, the arRNA comprises one or more chemical modifications. In some embodiments, the chemical modifications comprise methylation and/or phosphorothioation, e.g., 2'-O-methylation (2'-O-Me) and/or an internucleotide phosphorothioate bond. In certain embodiments, the first and last 3 or 5 nucleotides in the arRNA comprise 2'-O-Me modifications, and/or the linkages between the first and last 3, 4 or 5 nucleotides comprise phosphorothioate bonds. In certain embodiments, one or more or all uridines in the arRNA comprise 2'-O-Me modifications. In certain embodiments, a targeting nucleoside in the arRNA, and/or a nucleoside (e.g., one or two nucleosides directly adjacent to the 5'-end and/or the 3'-end of the targeting nucleoside) adjacent to the 5'-end and/or the 3'-end of the targeting nucleoside comprise 2'-O-Me modifications. In certain embodiments, a targeting nucleoside in the arRNA, and/or a nucleoside (e.g., one or two nucleosides directly adjacent to the 5'-end and/or the 3'-end of the targeting nucleoside) adjacent to the 5'-end and/or the 3'-end of the targeting nucleoside comprise 3'-phosphorothioate bond modifications. In certain embodiments, the arRNA does not comprise any chemical modification.

The present application further provides a virus vector, plasmid or linear nucleic acid chain, which comprises any arRNA described above according to the present application, and the arRNA does not comprise any chemical modification. The present application further provides a library, which comprises any arRNA described above according to the present application, or any virus vector, plasmid or linear nucleic acid chain described above according to the present application. The present application further provides a composition, which comprises any arRNA described above according to the present application, or any virus vector, plasmid or linear nucleic acid chain described above according to the present application. The present application provides a host cell, which comprises any arRNA described above according to the present application, or any virus vector, plasmid or linear nucleic acid chain described above according to the present application. In some embodiments, the host cell comprising any arRNA described above according to the present application is a eukaryocyte.

### BRIEF DESCRIPTION OF THE DRAWINGS

Fig. 1 shows the three-consecutive-base preference of the REPAIR system (Cox et al., 2017).
Fig. 2 shows the three-consecutive-base preference of the SNAP-ADAR system (Vogel et al., 2018).
Fig. 3 shows the three-consecutive-base preference of the LEAPER system (Qu et al., 2019).
Fig. 4 shows basic process of the LEAPER system, and an improvement of the present application.
Fig. 5 shows construction of 16 three-base motif reporter systems.
Fig. 6 shows design of 16 three-consecutive-complementary-base sequences, and the results corresponding to three-base motifs according to the design principle of arRNA in the LEAPER system in the prior art.
Fig. 7 shows first test results of the reporter system for the three-base motif of UAG.
Fig. 8 shows repeated experiment results of the reporter system for the three-base motif of UAG.
Fig. 9 shows test results of the reporter system for the three-base motif of UAG in the reference document related to the LEAPER system (Qu et al., 2019).
Fig. 10 shows test results of editing efficiencies for the three-base motif of UAG.
Fig. 11A to Fig. 11C show test results of editing efficiencies for three-base motifs of GAN, including GAU (Fig. 11A), GAG (Fig. 11B), and GAC (Fig. 11C).

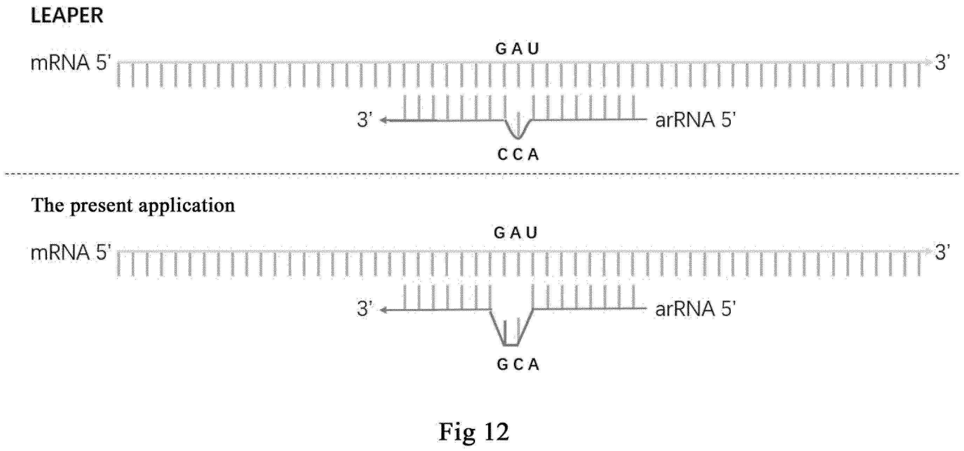

Fig. 12 shows improved arRNA design of the present application.
Fig. 13A to Fig. 13D show improved editing efficiencies of improved arRNA design of the present application for the three-base motifs of GAA (Fig. 13A), GAU (Fig. 13B), GAG (Fig. 13C), and GAC (Fig. 13D).
Fig. 14 shows a plasmid profile of Reporter 1 and the sequence thereof.
Fig. 15 shows test results of the C-to-U editing system, and in this figure, a target residue is C, and influences of changes in an upstream residue and the base opposite to the target C in three consecutive complementary bases on the editing efficiency are tested. In this figure, "/" indicates that no corresponding plasmid or arRNA is added, and only the same volume of water is added.
Fig. 16 shows repeated experiment results of some data in Fig. 15. In this figure, "/" indicates that no corresponding plasmid or arRNA is added, and only the same volume of water is added.
Fig. 17 shows test results of the C-to-U editing system. The test results are obtained under the conditions that three consecutive complementary bases in arRNA comprise a single mismatch corresponding to the target C, wherein the mismatched base corresponding to the target C is U, and the other two bases in the three consecutive complementary bases are completely matched with an upstream residue and a downstream residue that are adjacent to the target C.
Fig. 18 shows the data of the case where the three-base motif in mRNA is N*CA (as shown on the horizontal axis) and the three consecutive complementary bases in arRNA are GUU in Fig. 15, which are used for comparison with the data in Fig. 17.
Fig. 19A and Fig. 19B show analysis of the pairing results of the three-base motifs and the three consecutive complementary bases respectively in Fig. 18 and Fig. 17. Particularly, the three-base motifs and the three consecutive complementary bases in Fig. 19A are used to derive the results in Fig. 18, and the three-base motifs and the three consecutive complementary bases in Fig. 19B are used to derive the results in Fig. 17.
Fig. 20 shows comparison results of editing efficiencies of the reporter systems comprising multiple mismatches and a single mismatch, and the results are shown in %GFP. Particularly, the base paired with target residue C in the three-base motif is C, and the base opposite to the downstream residue adjacent to the target residue is U. In this figure, "5'-end base of mRNA" represents the upstream residue in the three-base motif. Other unmentioned bases in mRNA and arRNA are strictly complementarily paired.
Fig. 21 shows comparison results of the editing efficiencies of the reporter systems comprising multiple mismatches and a single mismatch, which are results shown in mean fluorescence intensity (MFI) of the same test in Fig. 20.
Fig. 22A to Fig. 22D show test results of the editing efficiencies of different designed arRNAs for the three-base motifs of ACA (Fig. 22A), TCA (Fig. 22B), CCA (Fig. 22C), and GCA (Fig. 22D).

### DETAILED DESCRIPTION OF THE APPLICATION

The present application provides a method for editing target RNA at a target residue position in a host cell, which comprises introducing ADAR-recruiting RNA (arRNA) or a construct encoding the arRNA into a host cell. The arRNA comprises a complementary RNA sequence hybridizing to a target RNA to form double-stranded RNA for recruiting a deaminase acting on RNA to deaminate a target residue in the target RNA, and the base type of the residue is changed after deamination. The present application provides a target RNA editing method, through the design of arRNA and the target RNA, the method significantly improves editing efficiencies of the three-base motifs other than UAG which are not in conformity with the natural preference of ADAR by using the ADAR-based RNA editing systems in the prior art, and breaks through the long-lasting limitation in selection of editing positions in the application of RNA editing. By the method of the present application, the scope and efficacy of treatment of diseases by RNA editing can be greatly expanded, so that more diseases (e.g., more inherited diseases) caused by gene mutations have the opportunity to be safely and effectively treated by RNA editing. By the method and/or arRNA of the present application, as for a disease caused by G->A mutation which may be treated in the future by RNA editing therapy, a three-base motif in which a mutation position is located can be selected more flexibly. For example, in a case that the three-base motif in which the mutation position is located is GAU, the editing efficiency of the prior art cannot meet the treatment requirements, while the editing efficiency of the method according to the present application is at least 10 times of that of the prior art. In addition, since the appropriately modified ADAR protein can perform RNA base editing of C->U, the method according to the present application can further improve editing efficiencies of an RNA editing system for different three-base motifs having a target residue of C.

Therefore, the present application provides a method for editing target RNA at a target residue position in a host cell, which comprises introducing ADAR-recruiting RNA (arRNA) or a construct encoding the arRNA into a host cell, wherein the arRNA comprises a complementary RNA sequence that hybridizes to the target RNA, and the target residue is located in a three-base motif comprising a 5' nearest-neighbor residue (upstream residue) of the target residue in the target RNA, the target residue, and a 3' nearest-neighbor residue (downstream residue)o of the target residue in the target RNA, wherein the three-base motif is not UAG, and the complementary RNA sequence comprises a mismatch directly opposite to the upstream residue and/or the downstream residue in the target RNA.

The "target RNA" herein is RNA to be edited. The "base" and "residue" herein refer to a nucleobase such as "adenine", "guanine", "cytosine", "thymine", "uracil", and "hypoxanthine". The term "adenosine", "guanosine", "cytidine", "thymidine", "uridine", and "inosine" refer to a nucleobase linked with the carbohydrate moiety of ribose or deoxyribose. The term "nucleoside" refers to a nucleobase linked with ribose or deoxyribose. The term "nucleotide" refers to the respective nucleobase-ribose-phosphate, or nucleobase-deoxyribose-phosphate. Sometimes, the terms adenosine and adenine (abbreviated as "A"), guanosine and guanine (abbreviated as "G"), cytosine and cytidine (abbreviated as "C"), uracil and uridine (abbreviated as "U"), thymine and thymidine (abbreviated as "T"), and inosine and hypoxanthine (abbreviated as "I") are interchangeable, and refer to a corresponding nucleobase, nucleoside or nucleotide. In a nucleic acid chain, the 3'-hydroxyl group of the previous nucleotide and 5'-phosphoric acid of the next nucleotide form a 3',5'-phosphodiester bond, a hydroxyl group -OH is removed from the 3'-end of a nucleotide, which is referred to as a "nucleotide residue" or "residue". Sometimes, the terms nucleobase, base, nucleoside, nucleotide, nucleotide residue, and residue are interchangeable, unless a difference is clearly described in the context.

As used herein, the "complementarity" of nucleic acids refers to the ability of a nucleic acid to form hydrogen bonds with another nucleic acid through conventional Watson-Crick base pairing. Percent complementarity represents the percentage of residues in a nucleic acid molecule that can form hydrogen bonds (i.e., Watson-Crick base pairing) with another nucleic acid molecule (e.g., about 5, 6, 7, 8, 9 or 10 out of 10 represents about 50%, 60%, 70%, 80%, 90% or 100% complementarity). "Completely complementary" refers to that all consecutive residues in a nucleic acid sequence form hydrogen bonds with the same number of consecutive residues in a second nucleic acid sequence. As used herein, "substantively complementary" refers to at least about 70%, 75%, 80%, 85%, 90%, 95%, 97%, 98%, 99% or 100% complementarity for a region of about 40, 50, 60, 70, 80, 100, 150, 200, 250 or more nucleotides, or refers to two nucleic acids hybridizing under strict conditions. For a single base or single nucleotide, according to the Watson-Crick base pairing principle, the paring of A with T or U, or the paring of C with G or I is referred to as complementary or matched, and vice versa. However, other base pairing is referred to as non-complementary or unmatched.

"Hybridization" refers to a reaction by which one or more polynucleotides form a complex, and the complex is stabilized by hydrogen bonds between bases of nucleotide residues. The hydrogen bonds may be formed by Watson Crick base pairing, Hoogstein binding or any other sequence-specific manner. A sequence that can hybridize to a given sequence is referred to as a "complementary sequence" of the given sequence.

The term "RNA editing" refers to phenomena, such as insertion, deletion, and substitution of bases on RNA. An enzyme usually used in many RNA editing systems is an adenosine deaminase acting on RNA (ADAR) or a variant thereof, or a complex comprising an ADAR functional domain. The ADAR protein family can bind to a double-stranded region of a specific RNA, and it can remove the -NH2 group from an adenosine (A) nucleotide base to convert A to inosine (I), and I is recognized as guanosine (G) and paired with cytidine (C) in the subsequent translation process of cells. The RNA editing of Adenosine-to-inosine (A->I) is the most common type of RNA editing in animals, and it is widely involved in several gene regulation mechanisms at the transcriptional and post-transcriptional levels, for example, it changes an amino acid sequence at the transcriptome level, and regulates the splicing and stability of mRNA and formation of circular RNA (Nishkura K. 2010). In mammalian cells, there are three types of ADAR proteins: ADAR1 (two isotypes: p110 and p150), ADAR2, and ADAR3 (without the catalytic activity). The researchers made λN peptide fuse with a human ADAR1 or ADAR2 deaminase domain to construct a λN-ADARDD system, which can be guided by fusion RNA consisting of BoxB stem-loop and antisense RNA so as to bind to a specific RNA target. According to this method, target A can be edited to I (A-C mismatch is introduced) at the target A base position to result in A-to-G RNA base editing. Other RNA editing methods comprises a method for editing target RNA by making antisense RNA fuse with the R/G motif (an ADAR-recruiting RNA scaffold) so as to overexpress ADAR1 or ADAR2 protein in mammalian cells, and a method for accurately targeting and editing RNA by using dCas13-ADAR. Editing at the RNA level not only avoids damage to the genome, but also can change the final biological functions.

The terms "deaminase-recruiting RNA", "dRNA", "arRNA" and "ADAR-recruiting RNA" are interchangeable herein, and refer to RNA that can recruit ADAR, an ADAR variant or certain complexes comprising an ADAR domain to deaminate target adenosine or target cytidine in RNA. In the context of the present application, the "target RNA" refers to that a deaminase-recruiting RNA sequence is designed as an RNA sequence completely complementary or substantively complementary to the target RNA, and the target RNA comprises a target residue. The "target residue" herein refers to a nucleotide residue to be modified by RNA editing such as introduction of ADAR enzyme and arRNA. A target sequence hybridizes to arRNA to form a double-stranded RNA (dsRNA) region comprising a target residue, which recruits an adenosine deaminase acting on the target residue (ADAR) or a variant thereof, and the enzyme or the variant thereof deaminates the target residue.

The "three-base motif" represents a three-consecutive-base sequence comprising a 5' nearest-neighbor residue (upstream residue) of a target residue in the target RNA, the target residue, and a 3' nearest-neighbor residue (downstream residue) of the target residue in the target RNA. In the context of the "three-base motif" according to the present application, the "target residue" is located at an "editing position", so the "target residue" and the "editing position" are interchangeable, unless otherwise specified. An upstream residue and a downstream residue in a three-base motif often determine the editing efficiency of RNA editing for a target residue. For example, editing efficiencies of RNA editing systems, such as REPAIR (WO2019005884A1), RESTORE (WO2020001793A1), and LEAPER (WO2020074001A1), for different three-base motifs are different, and this situation of different editing efficiencies for different three-base motifs is referred to as "three-consecutive-base preference" herein.

Three bases in the complementary RNA sequence which are directly opposite to the three-base motif in the target RNA,, i.e., a three-consecutive-base motif consisting of a base (referring to as a "targeting base" herein) directly opposite to the target residue, a 5' nearest-neighbor residue of the base, and a 3' nearest-neighbor residue of the base are referred to as "three consecutive complementary bases" herein.

Herein, all three-base motifs and three consecutive complementary bases are in the order of the 5'-end to the 3'-end.

In the method of the present application, the target RNA hybridizes to the arRNA to form a double-stranded RNA (dsRNA) region comprising the target residue, which recruits a deaminase acting on RNA, and this enzyme deaminates the target residue. The method of the present application comprises designing arRNA and introducing the arRNA or a construct encoding the arRNA into a host cell. A complementary RNA sequence in the arRNA sequence hybridizes to target RNA to form a double-stranded RNA capable of recruiting a deaminase acting on RNA so as to deaminate a target residue in the target RNA, and the base type of the residue can be changed after deamination. Due to deamination, adenosine (A) can be converted to inosine (I), and I is recognized as guanosine (G), thereby realizing A-to-G editing. Similarly, due to deamination, cytidine (C) can be converted to uridine (U), thereby realizing C-to-U editing.

The three-consecutive-base preference of RNA editing is shown, for example, in Fig. 2 and Fig. 3. The lower three-consecutive-base preference for three-base motifs having an upstream residue of guanosine (G) is a common feature of the current ADAR-based RNA editing methods. Similarly, in C-to-U editing, the published documents also reveal obvious three-consecutive-base preference. Due to the limitation of the three-consecutive-base preference, in order to meet practical application requirements and realize efficient editing, in the prior art it is necessary to select three-base motifs having higher three-consecutive-base preferences to edit in the deaminase-based RNA editing systems. Thus, the application scope of RNA editing is limited. The present application provides an improved method for editing target RNA at a target residue position in a host cell, which comprises introducing more mismatches to base positions directly opposite to a three-base motif in arRNA, which significantly improves editing efficiencies of the ADAR-based RNA editing systems in the prior art for the target bases in three-base motifs that do not meet the three-consecutive-base preference of the deaminase, thereby breaking through the long-lasting limitation in selection of editing positions in the application of RNA editing.

Therefore, in one aspect, the present application provides a method for editing target RNA at a target residue position in a host cell, which comprises introducing ADAR-recruiting RNA (arRNA) or a construct encoding the arRNA into a host cell, wherein the arRNA comprises a complementary RNA sequence, and the complementary RNA sequence hybridizes to target RNA to form double-stranded RNA capable of recruiting a deaminase acting on RNA so as to deaminate a target residue in the target RNA. The target residue is located in a three-base motif in the target RNA, and the three-base motif comprises a 5' nearest-neighbor residue (upstream residue) of the target residue in the target RNA, the target residue, and a 3' nearest-neighbor residue (downstream residue) of the target residue in the target RNA. A triplet formed by linking the upstream residue, the target residue, and the downstream residue in sequence in the order of the 5'-end to the 3'-end is referred to as a "three-base motif". In the present application, all three-base motifs are described in the order of the 5'-end to the 3'-end. The three bases (opposite to the three-base motif in the target RNA) in the complementary RNA sequence are also in the order of the 5'-end to the 3'-end.

The present application provides a method for editing target RNA at a target residue position in a host cell, which comprises introducing ADAR-recruiting RNA (arRNA) or a construct encoding the arRNA into a host cell, wherein the arRNA comprises a complementary RNA sequence that hybridizes to target RNA, and the target residue is located in a three-base motif comprising a 5' nearest-neighbor residue (upstream residue) of the target residue in the target RNA, the target residue, and a 3' nearest-neighbor residue (downstream residue) of the target residue in the target RNA; wherein the three-base motif is not UAG, and the complementary RNA sequence comprises a mismatch directly opposite to the upstream residue or the downstream residue in the target RNA.

In some embodiments, the present application provides a method for editing target RNA at a target residue position in a host cell, which comprises introducing ADAR-recruiting RNA (arRNA) or a construct encoding the arRNA into a host cell, wherein the arRNA comprises a complementary RNA sequence that hybridizes to the target RNA, and the target residue is located in a three-base motif comprising a 5' nearest-neighbor residue (upstream residue) of the target residue in the target RNA, the target residue, and a 3' nearest-neighbor residue (downstream residue) of the target residue in the target RNA, wherein the three-base motif is not UAG, and the complementary RNA sequence comprises mismatches directly opposite to the upstream residue and the downstream residue in the target RNA.

In certain embodiments, the upstream residue in the three-base motif is G. In certain embodiments, the upstream residue in the three-base motif is A. In certain embodiments, the upstream residue in the three-base motif is C. In certain embodiments, the downstream residue in the three-base motif is C. In certain embodiments, the downstream residue in the three-base motif is U. In certain embodiments, the downstream residue in the three-base motif is A. In certain embodiments, the three-base motif is selected from the group consisting of: GAG, GAC, GAA, GAU, AAG, AAC, AAA, AAU, CAG, CAC, CAA, CAU, UAA, UAC, and UAU. In certain embodiments, the three-base motif is GAU. In certain embodiments, the three-base motif is GAG. In certain embodiments, the three-base motif is GAA. In certain embodiments, the three-base motif is GAC. In some embodiments, the upstream residue in the target RNA is a nucleotide selected from the group consisting of: G, C, A and U, and the preferred order is as follows: G>C≈A>U. In some embodiments, the complementary RNA sequence comprises cytidine (C), adenosine (A) or uridine (U) directly opposite to the target adenosine in the target RNA. In some specific embodiments, the complementary RNA sequence comprises C directly opposite to the target adenosine in the target RNA.

According to the method of the present application, in some embodiments, the complementary RNA sequence that hybridizes to the target RNA further comprises one or more mismatches respectively opposite to a non-target adenosine in the target RNA. In certain embodiments, the mismatch nucleoside opposite to one or more non-target adenosine is guanosine. In some embodiments, the three-base motif is GAU, and three consecutive complementary bases (directly opposite to the three-base motif) comprised in the complementary RNA sequence are ACG or ACA. In some embodiments, the three-base motif is GAU, and three consecutive complementary bases (directly opposite to the three-base motif) comprised in the complementary RNA sequence are ACG. In some embodiments, the three-base motif is GAA, and three consecutive complementary bases (directly opposite to the three-base motif) comprised in the complementary RNA sequence are UCA, CCG, CCC or UCC. In certain embodiments, the three-base motif is GAA, and three consecutive complementary bases (directly opposite to the three-base motif) comprised in the complementary RNA sequence are UCA. In some embodiments, the three-base motif is GAC, and three consecutive complementary bases (directly opposite to the three-base motif) comprised in the complementary RNA sequence are GCG or GCA. In certain embodiments, the three-base motif is GAC, and three consecutive complementary bases (directly opposite to the three-base motif) comprised in the complementary RNA sequence are GCG. In some embodiments, the three-base motif is GAG, and three consecutive complementary bases (directly opposite to the three-base motif) comprised in the complementary RNA sequence are CCG, CCA, CCC, UCC or UCG. In certain embodiments, the three-base motif is GAG, and three consecutive complementary bases (directly opposite to the three-base motif) comprised in the complementary RNA sequence are CCG. In some embodiments, the upstream residue in the three-base motif is G, and the base opposite to the upstream residue in the complementary RNA is G or A. In some embodiments, the downstream residue in the three-base motif is strictly complementary to an opposite base in the complementary RNA. In some embodiments, the upstream residue in the three-base motif is G, wherein the base opposite to the upstream residue in the complementary RNA is G or A, and the downstream residue in the three-base motif is strictly complementary to an opposite base in the complementary RNA. In some embodiments, the complementary RNA sequence comprises C directly opposite to the target adenosine in the target RNA, the upstream residue in the three-base motif is G, wherein the base opposite to the upstream residue in the complementary RNA is G or A, and the downstream residue in the three-base motif is strictly complementary to an opposite base in the complementary RNA. In some embodiments, the complementary RNA sequence comprises C directly opposite to the target adenosine in the target RNA, the upstream residue in the three-base motif is G, wherein the base opposite to the upstream residue in the complementary RNA is G, and the downstream residue in the three-base motif is strictly complementary to an opposite base in the complementary RNA. The RNA editing efficiency of the method according to the present application is increased by at least 90% to 1100%, for example, at least 100%, 200%, 300%, 400%, 500%, 600%, 700%, 800%, 900%, and 1000%.

In some embodiments, the target adenosine (A) in the target RNA is deaminated through an adenosine deaminase acting on RNA (ADAR). In certain embodiments, the adenosine deaminase is natural ADAR or a homologous protein thereof. In certain embodiments, the adenosine deaminase is an adenosine deaminase functional variant that is modified but retains the adenosine deaminase activity, for example, a variant that is obtained by modifying natural ADAR or a homologous protein thereof with one or more position mutations and retains the adenosine deaminase activity. In certain embodiments, the adenosine deaminase is a fusion protein comprising an ADAR catalytic domain, or ADAR homologous protein catalytic domain, or adenosine deaminase functional variant. In certain embodiments, the fusion protein comprising an ADAR protein catalytic domain is a fusion protein comprising Cas13 protein that loses catalytic activity after mutation and an ADAR functional domain, or ADAR homologous protein functional domain, or adenosine deaminase functional variant. In some embodiments, the deaminase with the adenosine deaminase activity is exogenously introduced into the host cell or expressed in the host cell by introducing a construct encoding the deaminase. In certain embodiments, the fusion protein comprising an ADAR protein catalytic domain is a fusion protein comprising λN peptide and an ADAR functional domain, or ADAR homologous protein catalytic domain, or adenosine deaminase functional variant. In certain embodiments, the fusion protein comprising an ADAR protein catalytic domain is SNAP-tag-labelled ADAR, or a SNAP-tag-labelled ADAR functional variant. In certain embodiments, the ADAR is ADAR1 and/or ADAR2. In some embodiments, ADAR is one or more ADAR selected from the group consisting of: hADAR1, hADAR2, mouse ADAR1, and mouse ADAR2.

In certain embodiments, the ADAR is expressed by the host cell. In certain embodiments, ADAR naturally or endogenously occurs in a host cell, for example, ADAR naturally or endogenously occurs in a eukaryocyte. In certain embodiments, the ADAR protein is exogenously introduced into the host cell. In certain embodiments, the ADAR or a construct encoding the ADAR is introduced into a host cell. In some embodiments, the construct comprises, but is not limited to, a linear nucleic acid, a plasmid, a vector, etc. In the above method, the ADAR comprises the natural ADAR and the homologous protein thereof, the adenosine deaminase functional variant that is modified but retains the adenosine deaminase activity (e.g., a variant that is obtained by modifying natural ADAR or a homologous protein thereof with one or more position mutations and retains the adenosine deaminase activity), or the fusion protein comprising an ADAR catalytic domain, or ADAR homologous protein catalytic domain, or adenosine deaminase functional variant. In certain embodiments, the fusion protein comprising an ADAR catalytic domain or ADAR homologous protein catalytic domain or adenosine deaminase functional variant is a fusion protein comprising a targeting domain and the ADAR catalytic domain or ADAR homologous protein catalytic domain or adenosine deaminase functional variant. In certain embodiments, the targeting domain comprises any one of, but is not limited to: Cas13 protein that loses catalytic activity after mutation, λN peptide, and SNAP-tag. In some embodiments, ADAR is one or more ADAR selected from the group consisting of: hADAR1, hADAR2, mouse ADAR1, and mouse ADAR2. In some embodiments, the method does not comprise the step of introducing any protein into a host cell. In certain embodiments, the ADAR is ADAR1 and/or ADAR2.

In another aspect, the present application provides a method for editing target RNA at a target residue position in a host cell, which comprises introducing ADAR-recruiting RNA (arRNA) or a construct encoding the arRNA into a host cell, wherein the arRNA comprises a complementary RNA sequence that hybridizes to target RNA, wherein the target residue is located in a three-base motif comprising a 5' nearest-neighbor residue (upstream residue) of the target residue in the target RNA, the target residue, and a 3' nearest-neighbor residue (downstream residue) of the target residue in the target RNA, and the target residue is cytidine (C); wherein the complementary RNA sequence comprises a mismatch directly opposite to the upstream residue and/or the downstream residue in the target RNA, and the method further comprises introducing a deaminase with the cytidine deaminase activity or a cytidine deaminase or a construct encoding the deaminase into a host cell. In some embodiments, the deaminase with the cytidine deaminase activity is a deaminase with the C-to-U catalytic activity that is obtained by gene modification of ADAR protein or a fusion protein comprising an ADAR catalytic domain. In some embodiments, the deaminase with the cytidine deaminase activity further comprises a targeting domain.

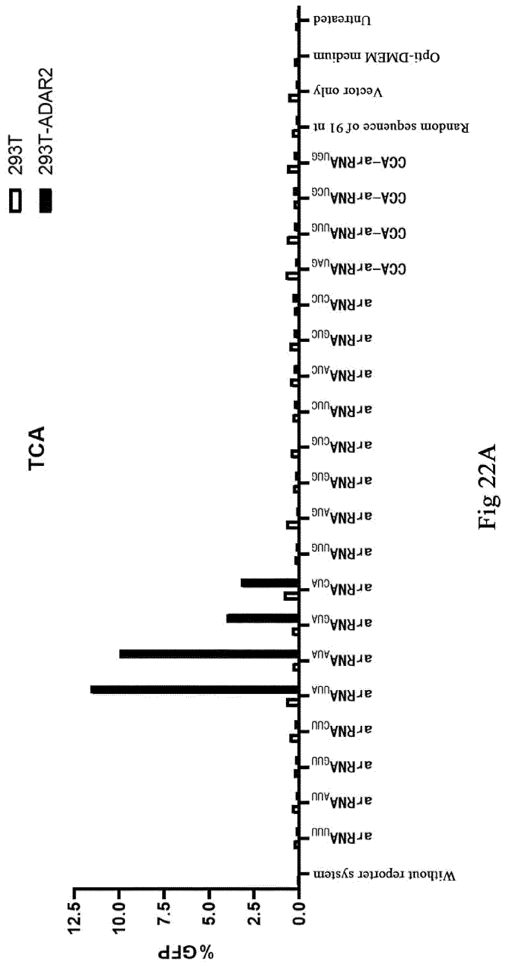

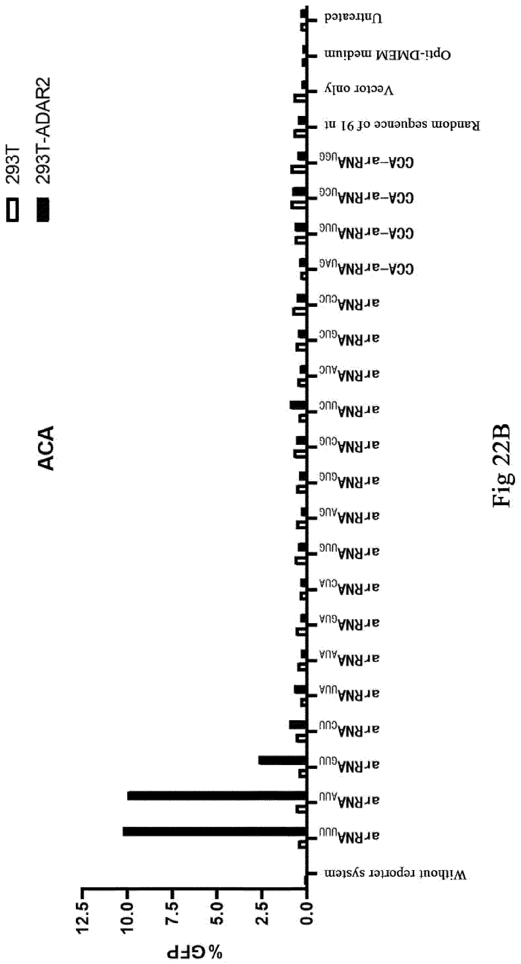

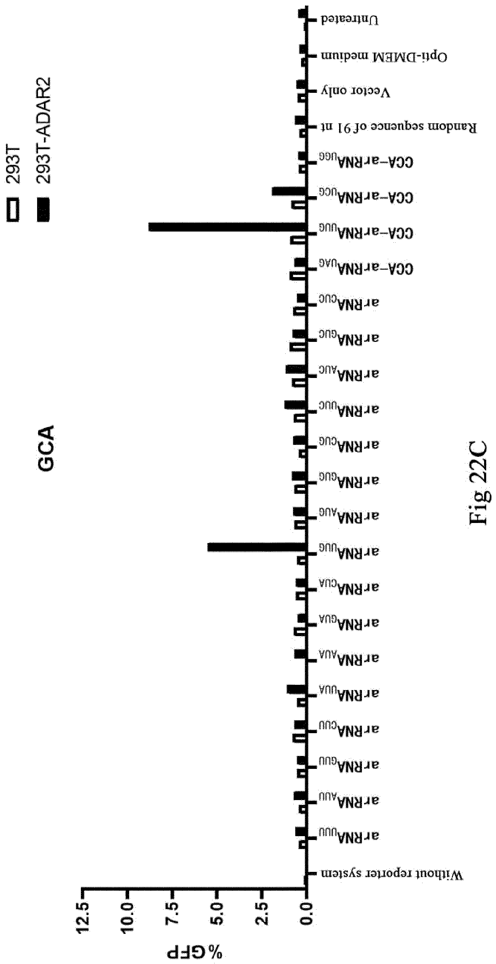

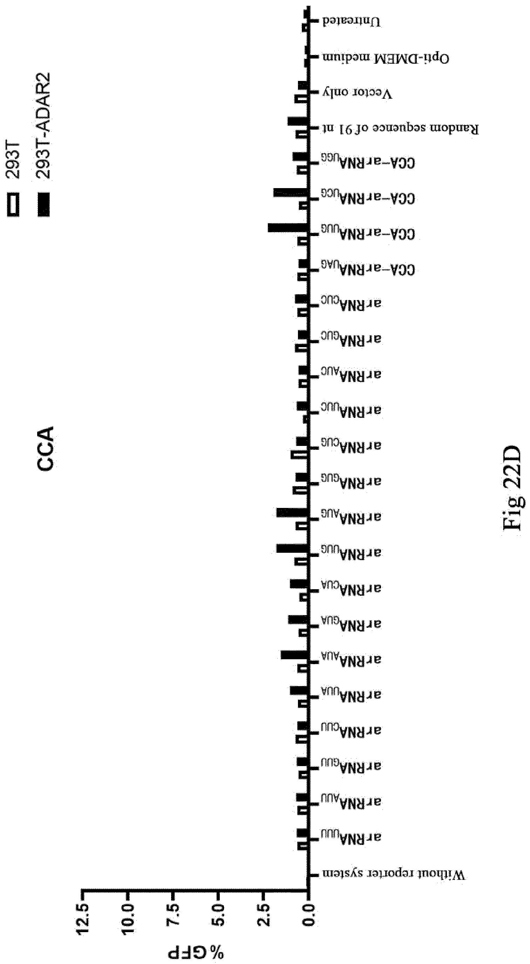

In some embodiments, the three-base motif in which the target cytidine is located is any one selected from the group consisting of: GCG, GCC, GCA, GCU, ACG, ACC, ACA, ACU, CCG, CCC, CCA, CCU, UCA, UCC, UCU, and UCG. In some embodiments, the arRNA comprises an unpaired nucleotide at a position corresponding to the target residue in the target RNA to form a mismatch with the target residue. In some embodiments, the complementary RNA sequence in the arRNA that can hybridize to the target RNA comprises cytidine, adenosine or uridine directly opposite to the target cytidine in the target RNA. In certain embodiments, the complementary RNA sequence comprises cytidine directly opposite to the target cytidine. In certain embodiments, the arRNA comprises one or more unpaired nucleotides at positions corresponding to non-target editing positions in the target RNA to form one or more mismatches with the non-target positions in the target RNA. Cytidine-to-uridine editing efficiencies in a case that there is a single mismatch opposite to the target residue in the three-base motif and in a case that there are multiple mismatches of the residues in the three-base motif are respectively tested in Example 4, and results are shown in Fig. 22. It can be seen that, in a case that the upstream residue in the three-base motif is A or U, the editing efficiency in a case that there are multiple mismatches is equal to the editing efficiency in a case that there is a single mismatch of the target residue, while in a case that the upstream residue in the three-base motif is G, the editing efficiency in a case that there is a single mismatch of the target residue is very low, and the C-to-U editing efficiency can be significantly improved by introducing more mismatches. Therefore, in some embodiments, the upstream residue in the three-base motif is G, and the complementary RNA sequence comprises G directly opposite to the upstream residue. In some embodiments, the three-base motif is ACA, and the complementary RNA sequence comprises AUU or GUU opposite to the three-base motif. In some embodiments, the three-base motif is ACA, and the complementary RNA sequence preferably comprises AUU opposite to the three-base motif. In some embodiments, the three-base motif is UCA, and the complementary RNA sequence comprises AUA, GUA or CUA opposite to the three-base motif. In some embodiments, the three-base motif is UCA, and the complementary RNA sequence preferably comprises AUA opposite to the three-base motif. In certain embodiments, the three-base motif is GCA, and the complementary RNA sequence comprises UUG or UCG opposite to the three-base motif. In some embodiments, the three-base motif is GCA, and the complementary RNA sequence preferably comprises UUG opposite to the three-base motif. In some embodiments, the three-base motif is CCA, and the complementary RNA sequence comprises AUG opposite to the three-base motif. In certain embodiments, the target residue in the three-base motif in the target RNA is cytidine, the upstream residue in the three-base motif is a nucleotide selected from G, C, A, and U, and the preferred order is as follows: G>C>A≈U.

In some embodiments, the arRNA recruits a deaminase with the cytidine deaminase activity to the target RNA so as to deaminate and convert the target cytidine (C) in the target RNA to uridine. The cytidine deaminase is an cytidine deaminase or a homologous protein variant thereof that has the cytidine deaminating activity after modification (e.g., deletion or mutation of amino acids at one or more positions). In certain embodiments, the cytidine deaminase that has the cytidine deaminating activity after modification comprises those disclosed in the prior art, for example, one or more mutant cytidine deaminase fragments with the cytidine deaminating activity that are disclosed in Abudayyeh et al., 2019. In certain embodiments, the cytidine deaminase that has the cytidine deaminating activity after modification is ADAR2 comprising one or more mutant selected from: E488Q/V351 G/S486A/T37 5S/S370C/P462A/N 597I/L332I/I398V IK350I/M383L/D619G/S582T/V 440I/S495N/K418E/S661T. In certain specific embodiments, the cytidine deaminase that has the cytidine deaminating activity after modification is ADAR2 comprising all of the following mutants: E488Q/V351 G/S486A/T37 5S/S370C/P462A/N 597I/L332I/I398V IK350I/M383L/D619G/S582T/V 440I/S495N/K418E/S661T. In certain embodiments, the cytidine deaminase is a fusion protein of ADAR2 catalytic domain comprising all of the following mutants: E488Q/V351 G/S486A/T37 5S/S370C/P462A/N 597I/L332I/I398V IK350I/M383L/D619G/S582T/V 440I/S495N/K418E/S661T. In some embodiments, the deaminase with the cytidine deaminase activity further comprises a targeting domain. In certain embodiments, the targeting domain comprises any one of, but is not limited to: Cas13 protein that loses catalytic activity after mutation, λN peptide, and SNAP-tag.

In certain embodiments, the method comprises introducing the cytidine deaminase or the fusion protein or a construct encoding the cytidine deaminase or the fusion protein into a host cell. In certain embodiments, the construct comprises, but is not limited to: a linear nucleic acid, a plasmid, a vector, etc.

According to the above method of the present application, the arRNA is single-stranded RNA. In some embodiments, the complementary RNA sequence is completely single-stranded. In certain embodiments, the arRNA comprises one or more (e.g., 1, 2, 3 or more) double-stranded regions, and/or one or more stem-loop regions. In certain embodiments, the arRNA is composed of the complementary RNA sequence only.

According to the method of the present application, in some embodiments, the complementary RNA sequence comprises two or more mismatches corresponding to the target sequence. In some embodiments, the complementary RNA sequence comprises one or more mismatches corresponding to the target sequence in addition to the three consecutive complementary bases. In some embodiments, when the complementary RNA sequence hybridizes to the target sequence, one or more wobble base pairs may be formed. In some embodiments, when the complementary RNA sequence hybridizes to the target sequence, one or more unilateral protrusions may be formed. In some embodiments, when the complementary RNA sequence hybridizes to the target sequence, one or more wobble base pairs and one or more unilateral protrusions may be formed.

According to the method of the present application, in some embodiments, the arRNA has a length of about 20-260 nucleotides, for example, the arRNA has a length less than or equal to any one of about 30, 40, 45, 50, 55, 60, 65, 70, 75, 80, 85, 90, 95, 100, 105, 110, 120, 130, 140, 150, 160, 170, 180, 190, 200, or more nucleotides. In certain embodiments, the complementary RNA sequence has a length of any one of 40-260, 45-250, 50-240, 60-230, 65-220, 70-220, 70-210, 70-200, 70-190, 70-180, 70-170, 70-160, 70-150, 70-140, 70-130, 70-120, 70-110, 70-100, 70-90, 70-80, 75-200, 80-190, 85-180, 90-170, 95-160, 100-200, 100-150, 100-175, 110-200, 110-175, 110-150, or 105-140 nucleotides. In some embodiments, the arRNA has a length of about 60-200 (e.g., any one of about 60-150, 65-140, 68-130, or 70-120) nucleotides. In some embodiments, the arRNA further comprises an ADAR-recruiting domain.

According to the method of the present application, in some embodiments, the arRNA comprises one or more chemical modifications. In some embodiments, the chemical modifications comprise methylation and/or phosphorothioation, e.g., 2'-O-methylation (2'-O-Me) and/or an internucleotide phosphorothioate bond. In certain embodiments, the first and last 3 or 5 nucleotides in the arRNA comprise 2'-O-Me modifications, and/or the linkages between the first and last 3 or 5 nucleotides comprise phosphorothioate bond modifications. In certain embodiments, one or more or all uridines in the arRNA comprise 2'-O-Me modifications. In certain embodiments, a targeting nucleoside in the arRNA and/or a nucleoside (e.g., one or two nucleosides directly adjacent to the 5'-end and/or the 3'-end of the targeting nucleoside) adjacent to the 5'-end and/or the 3'-end of the targeting nucleoside comprise 2'-O-Me modifications. In certain embodiments, a targeting nucleoside in the arRNA and/or a nucleoside (e.g., one or two nucleosides directly adjacent to the 5'-end and/or the 3'-end of the targeting nucleoside) adjacent to the 5'-end and/or the 3'-end of the targeting nucleoside comprise 3'-phosphorothioate bond modifications. In certain embodiments, the arRNA does not comprise any chemical modification.

According to the method of the present application, in some embodiments, the target RNA is RNA selected from messenger RNA precursor, messenger RNA, ribosome RNA, transfer RNA, long non-coding RNA, and small RNA. In some embodiments, according to the method of the present application, the target residue in the target RNA is edited to result in a missense mutation, premature termination codon, aberrant splicing or alternative splicing in the target RNA, alternatively, to reverse a missense mutation, premature termination codon, aberrant splicing or alternative splicing in the target RNA. In some embodiments, according to the method of the present application, the target residue in the target RNA is edited to result in point mutation, truncation, extension and/or misfolding of a protein encoded by the target RNA, alternatively, to obtain a functional, full-length, correctly folded and/or wild-type protein by reversing a missense mutation, premature termination codon, aberrant splicing or alternative splicing in the target RNA.

According to the method of the present application, in some embodiments, the host cell is a eukaryocyte. In certain embodiments, the host cell is a mammalian cell. In certain embodiments, the host cell is a human or mouse cell.

By any method for editing target RNA at a target residue position in a host cell according to the present application, edited RNA or a host cell comprising the edited RNA can be produced. Therefore, the present application further provides edited RNA or a host cell comprising the edited RNA produced by the target RNA editing method according to the present application.

The method for editing target RNA at a target residue position in a host cell according to the present application can be applied to treat or prevent a disease or condition in an individual. Therefore, the present application further provides a method for treating or preventing a disease or condition in an individual, which comprises editing the target RNA associated with a disease or condition in an individual cell by any method for editing target RNA at a target residue position in a host cell described above according to the present application. In some embodiments, the disease or condition is an inherited gene disease, or a disease or condition associated with one or more acquired gene mutations (e.g., drug resistance).

The present application further provides RNA (arRNA) that can be applied to the method of the present application for recruiting a deaminase acting on RNA so as to deaminate a target residue in target RNA, which comprises a complementary RNA sequence that hybridizes to the target RNA, wherein the target residue is located in a three-base motif comprising a 5' nearest-neighbor residue (upstream residue) of the target residue in the target RNA, the target residue, and a 3' nearest-neighbor residue (downstream residue) of the target residue in the target RNA, wherein the three-base motif is not UAG, and the complementary RNA sequence comprises a mismatch directly opposite to the upstream residue and/or the downstream residue in the target RNA.

According to the arRNA of the present application, in some embodiments, the target residue (targeted by the arRNA) in the three-base motif in the target RNA is adenosine, wherein the upstream residue in the target RNA is a nucleotide selected from G, C, A, and U, and the preferred order is as follows: G>C≈A>U. In some embodiments, the three-base motif is selected from the group consisting of: GAG, GAC, GAA, GAU, AAG, AAC, AAA, AAU, CAG, CAC, CAA, CAU, UAA, UAC, and UAU. In certain embodiments, the arRNA comprises cytidine (C), adenosine (A) or uridine (U) directly opposite to the target adenosine in the target RNA. In some specific embodiments, the arRNA comprises C directly opposite to the target adenosine in the target RNA. In certain embodiments, the arRNA that hybridizes to the target RNA further comprises one or more mismatches respectively opposite to a non-target adenosine in the target RNA. In certain embodiments, the mismatche opposite to one or more non-target adenosine is guanosine. In some embodiments, the upstream residue in the three-base motif is G, and the base opposite to the upstream residue in the complementary RNA is G or A. In certain embodiments, the three-base motif is GAU, and three consecutive complementary bases (directly opposite to the three-base motif) comprised in the arRNA are ACG or ACA. In certain embodiments, the three-base motif is GAU, and three consecutive complementary bases (directly opposite to the three-base motif) comprised in the arRNA are ACG. In certain embodiments, the three-base motif is GAA, and three consecutive complementary bases (directly opposite to the three-base motif) comprised in the arRNA are UCA, CCG, CCC or UCC. In certain embodiments, the three-base motif is GAA, and three consecutive complementary bases (directly opposite to the three-base motif) comprised in the arRNA are UCA. In certain embodiments, the three-base motif is GAC, and three consecutive complementary bases (directly opposite to the three-base motif) comprised in the arRNA are GCG or GCA. In certain embodiments, the three-base motif is GAC, and three consecutive complementary bases (directly opposite to the three-base motif) comprised in the arRNA are GCG. In certain embodiments, the three-base motif is GAG, and three consecutive complementary bases (directly opposite to the three-base motif) comprised in the arRNA are CCG, CCA, CCC, UCC or UCG. In certain embodiments, the three-base motif is GAG, and three consecutive complementary bases (directly opposite to the three-base motif) comprised in the arRNA are CCG. In certain embodiments, the arRNA comprises one or more mismatches respectively opposite to a non-target adenosine in the target RNA.

According to the arRNA of the present application, in some embodiments, the target residue (targeted by the arRNA) in the three-base motif in the target RNA may be cytidine (C), which is referred to as target cytidine. In certain embodiments, the upstream residue in the three-base motif is a nucleotide selected from G, C, A, and U, and the preferred order is as follows: G>C>A≈U. In certain embodiments, the three-base motif in which the target cytidine is located is any one selected from the group consisting of: GCG, GCC, GCA, GCU, ACG, ACC, ACA, ACU, CCG, CCC, CCA, CCU, UCA, UCC, UCU, and UCG. In certain embodiments, the upstream residue in the three-base motif is G, and the base opposite to the upstream residue in the complementary RNA is G. In some embodiments, the downstream residue in the three-base motif is A, and the base opposite to the downstream residue in the complementary RNA is U or A. In some embodiments, the three-base motif is ACA, and the complementary RNA sequence comprises AUU or GUU opposite to the three-base motif. In some embodiments, the three-base motif is ACA, and the complementary RNA sequence comprises AUU opposite to the three-base motif. In some embodiments, the three-base motif is UCA, and the complementary RNA sequence comprises AUA, GUA or CUA opposite to the three-base motif. In some embodiments, the three-base motif is UCA, and the complementary RNA sequence comprises AUA opposite to the three-base motif. In some embodiments, the three-base motif is GCA, and the complementary RNA sequence comprises UUG or UCG opposite to the three-base motif. In some embodiments, the three-base motif is GCA, and the complementary RNA sequence comprises UUG opposite to the three-base motif. In some embodiments, the three-base motif is CCA, and the complementary RNA sequence comprises AUG opposite to the three-base motif. In certain embodiments, the arRNA comprises an unpaired nucleotide at a position corresponding to the target residue in the target RNA to form a mismatch with the target residue. In certain embodiments, the complementary RNA sequence in the arRNA that can hybridize to the target RNA comprises cytidine, adenosine or uridine directly opposite to the target cytidine in the target RNA. In certain embodiments, the complementary RNA sequence comprises cytidine directly opposite to the target cytidine. In certain embodiments, the arRNA comprises one or more unpaired nucleotides at positions corresponding to non-target editing positions in the target RNA to form one or more mismatches with the non-target positions in the target RNA.

According to the arRNA of the present application, in some embodiments, the arRNA is single-stranded RNA. In some embodiments, the complementary RNA sequence is completely single-stranded. In certain embodiments, the arRNA comprises one or more (e.g., 1, 2, 3 or more) double-stranded regions and one or more stem-loop regions. In certain embodiments, the arRNA comprises one or more (e.g., 1, 2, 3 or more) double-stranded regions. In certain embodiments, the arRNA comprises one or more (e.g., 1, 2, 3 or more) stem-loop regions. In certain embodiments, the arRNA comprises a region that can form an intramolecular stem-loop structure for recruiting ADAR enzyme. In certain embodiments, the arRNA does not comprise a region that can form an intramolecular stem-loop structure for recruiting ADAR enzyme. In certain embodiments, the arRNA is composed of the complementary RNA sequence only.

According to the arRNA of the present application, in some embodiments, when the complementary RNA sequence hybridizes to the target sequence, one or more wobble base pairs may be formed. In some embodiments, when the complementary RNA sequence hybridizes to the target sequence, one or more unilateral protrusions may be formed. In some embodiments, when the complementary RNA sequence hybridizes to the target sequence, one or more wobble base pairs and one or more unilateral protrusions may be formed.

According to the arRNA of the present application, in some embodiments, the arRNA has a length of about 20-260 nucleotides, for example, the arRNA has a length less than or equal to any one of about 30, 40, 45, 50, 55, 60, 65, 70, 75, 80, 85, 90, 95, 100, 105, 110, 120, 130, 140, 150, 160, 170, 180, 190, 200, or more nucleotides. In certain embodiments, the complementary RNA sequence has a length of any one of 40-260, 45-250, 50-240, 60-230, 65-220, 70-220, 70-210, 70-200, 70-190, 70-180, 70-170, 70-160, 70-150, 70-140, 70-130, 70-120, 70-110, 70-100, 70-90, 70-80, 75-200, 80-190, 85-180, 90-170, 95-160, 100-200, 100-150, 100-175, 110-200, 110-175, 110-150, or 105-140 nucleotides. In some embodiments, the arRNA has a length of about 60-200 (e.g., any one of about 60-150, 65-140, 68-130, or 70-120) nucleotides. In some embodiments, the arRNA further comprises an ADAR-recruiting domain.

According to the arRNA of the present application, in some embodiments, the arRNA comprises one or more chemical modifications. In some embodiments, the chemical modifications comprise methylation and/or phosphorothioation, e.g., 2'-O-methylation (2'-O-Me) and/or an internucleotide phosphorothioate bond. In certain embodiments, the first and last 3 or 5 nucleotides in the arRNA comprise 2'-O-Me modifications, and/or the linkages between the first and last 3 or 5 nucleotides comprise phosphorothioate bond modifications. In certain embodiments, one or more or all uridines in the arRNA comprise 2'-O-Me modifications. In certain embodiments, a targeting nucleoside in the arRNA and/or a nucleoside (e.g., one or two nucleosides directly adjacent to the 5'-end and/or the 3'-end of the targeting nucleoside) adjacent to the 5'-end and/or the 3'-end of the targeting nucleoside comprise 2'-O-Me modifications. In certain embodiments, a targeting nucleoside in the arRNA and/or a nucleoside (e.g., one or two nucleosides directly adjacent to the 5'-end and/or the 3'-end of the targeting nucleoside) adjacent to the 5'-end and/or the 3'-end of the targeting nucleoside comprise 3'-phosphorothioate bond modifications. In certain embodiments, the arRNA does not comprise any chemical modification.

The present application further provides a virus vector, plasmid or linear nucleic acid chain, which comprises any arRNA described above according to the present application, and the arRNA does not comprise any chemical modification. The present application further provides a library, which comprises any arRNA described above according to the present application, or any virus vector, plasmid or linear nucleic acid chain described above according to the present application. The present application further provides a composition, which comprises any arRNA described above according to the present application, or any virus vector, plasmid or linear nucleic acid chain described above according to the present application. The present application further provides a host cell, which comprises any arRNA described above according to the present application, or any virus vector, plasmid or linear nucleic acid chain described above according to the present application. In some embodiments, the host cell comprising any arRNA described above according to the present application is a eukaryocyte.

### EXAMPLES

Referring to the technical route of LEAPER (WO2020074001A1), a small arRNA fragment partially or completely complementary to target RNA comprising target adenosine (A) is exogenously introduced, and the RNA is used for recruiting endogenous ADAR to perform A-to-I editing on the target A. The arRNA is synthesized in vitro, and has a length of 71-111 nt. As shown in Fig. 4, compared with the ADAR protein-recruiting RNA used in the existing editing technologies by using ADAR protein or an ADAR functional domain (such as LEAPER), three bases (directly opposite to a three-base motif in a target sequence) in arRNA used in the present application have weaker complementarity to the three-base motif, i.e., in addition to a mismatch opposite to the target A, the three bases (directly opposite to the three-base motif) in the arRNA further comprise a base mismatched with an upstream residue and/or a downstream residue. Such a change breaks through the three-consecutive-base preference, such that freer and more efficient editing can be performed on three-base motifs having an upstream residue of G or the three-base motifs other than UAG by the existing and future ADAR-based editing methods.

### Example 1: Construction of three-base motif reporter systems and corresponding arRNAs

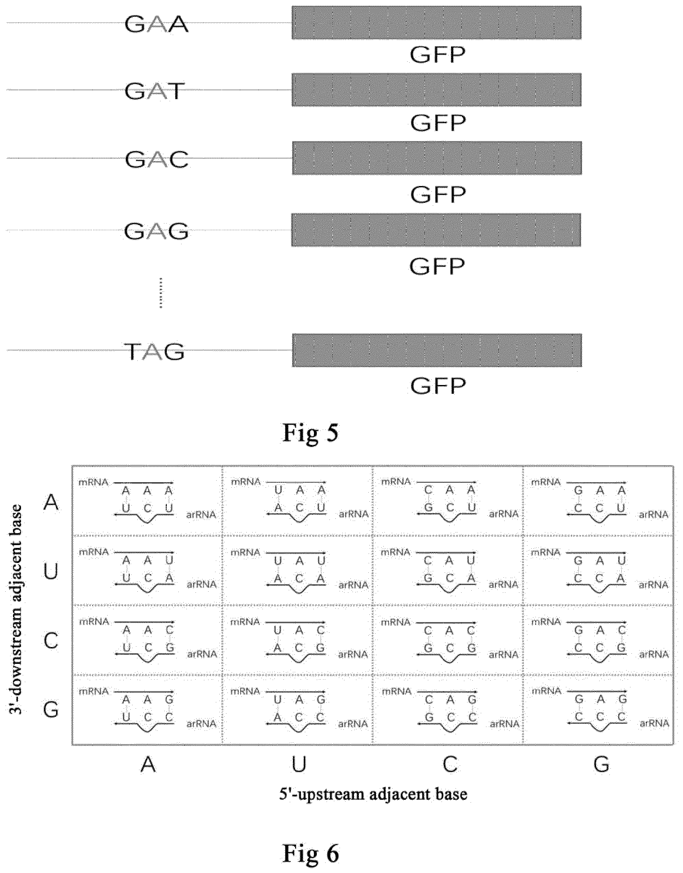

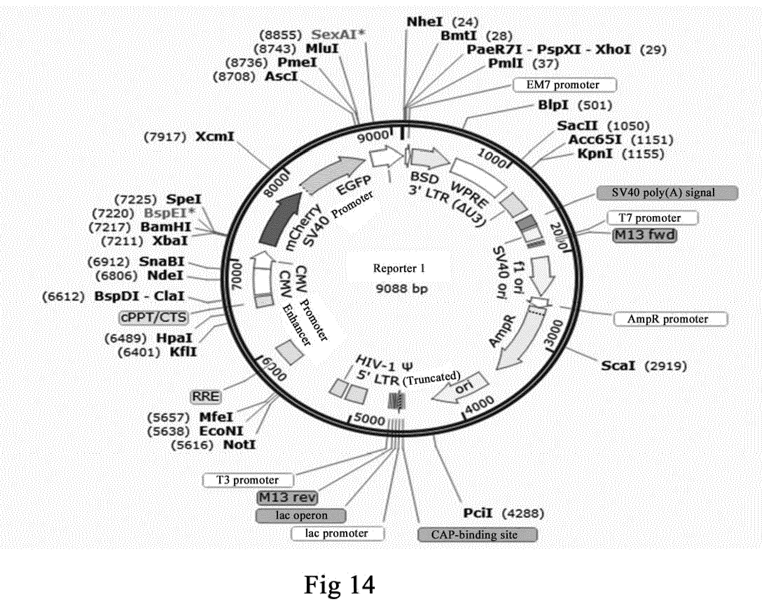

Firstly, reporter systems comprising 16 three-base motifs were constructed. In the document related to LEAPER, a difference between editing efficiencies for the three-base motif of UAG is tested (Qu et al., 2019). In the present example, in order to maintain the consistency of the control, nucleosides that can be complementarily paired with arRNA except for the editing position were designed to be the same as sequences in the above document related to LEAPER, as shown in Fig. 5. An original plasmid Reporter 1 was donated by Professor Wei Wensheng of School of Life Sciences, Peking University. A plasmid profile is shown in Fig. 14, and the plasmid comprises a sequence shown in Table 4. Primers related to 16 three-base motifs shown in Table 1 were synthesized, and PCR amplification (Q5^{®} High-Fidelity 2X Master Mix, NEB M0492L), enzyme digestion (XbaI, NEB R0145L; AscI, NEB R0558L), recovery with agarose gel (Seakem LE agarose, Lonza 5502; GeneJET Gel Extraction and DNA Cleanup Micro Kit, Thermo Fisher K0832), and assembly (NEBuilder^{®} HiFi DNA Assembly Master Mix, NEB E2621L) were performed by the method well-known to researchers in the art and described in J. Sambrook and M. R. Green, Molecular Cloning: a Laboratory Manual (4th Edition, 2017) and using materials shown in Table 2, and the amplification product was assembled into Reporter 1 to replace the original target RNA coding sequence in Reporter 1, then the Reporter 1 was transformed into competent cells (Trans1-T1 Phage Resistant Chemically Competent Cell, TransGen CD501-02), and clones were picked for sequencing the next day. Plasmids were extracted from clones with correct sequencing results, and packaged as lentiviruses. 293T cells were infected with these lentiviruses packaged with different three-base motif encoding genes, respectively. After infection for 48h, 16 types of 293T cells that can respectively transcribe mRNA (target RNA) comprising different three-base motifs were obtained, i.e., final three-base motif reporter system cells, which were named as three-base motifs shown in Table 2.

In order to detect whether a mismatch (opposite to an upstream residue and/or a downstream residue in a three-base motif) in arRNA can improve the editing efficiency for a specific three-base motif, 16 types of arRNAs were chemically synthesized in the present example, and the design principle was as follows: single-stranded RNA reversely complementary to an RNA fragment comprising 3'-downstream 55 nt and 5'-upstream 25 nt adjacent to the target A in a three-base motif in mRNA was taken, wherein the base opposite to the target A in the three-base motif was C. In a case that other bases in arRNA were kept unchanged, bases corresponding to an upstream residue and a downstream residue were respectively selected from A, C, G, and U, and 16 (4×4=16) types of arRNAs were obtained by combining the 4 bases corresponding to the upstream residue and the other 4 bases corresponding to the downstream residue. Specific sequences are shown in Table 3.

### Example 2: Comparison of the editing efficiencies of different arRNAs for the three-base motif of UAG based on proportions of GFP-positive cells

As shown in Fig. 5, each of the 16 types of target RNAs in Example 1 has a green fluorescent protein (GFP) nucleic acid sequence at the 3'-end of the target sequence. The sequence can be translated correctly into a GFP emitting a green fluorescence under normal conditions. However, in a case that the three-base motif is UAG, because UAG is a termination codon, translation will be terminated at this position, and the sequence cannot be translated into GFP. In the present example, the LEAPER system was used to edit A in the three-base motif of UAG. If A is successfully edited, UAG will be converted to UIG, UIG will be recognized as UGG in the translation process, so that translation will be not terminated and the downstream GFP can be normally translated. Therefore, according to proportions of GFP-positive cells, editing efficiencies of different arRNAs can be roughly determined.

In all tests in the present study, an RNAiMAX reagent (Invitrogen 13778150) was used to transfect the 16 types of arRNAs of Example 1 into cells respectively, and the specific procedure was as follows:
I. Cells were cultured in DMEM (Hyclone SH30243.01) containing 10% FBS (Vistech SE100-011). The reporter system cells were transferred to a 12-well plate at a density of 150000 cells/well. This time point was denoted as 0 hour.
II. 24h after cell passage, 12.5 pmol of arRNA was transferred to each well by using an RNAiMAX reagent (Invitrogen 13778150). Transfection was performed according to supplier's instructions.
III. 72h after cell passage, the cells in each well were digested by using a pancreatin (Invitrogen 25300054), and the FITC channel intensity was analyzed by using a flow cytometer.

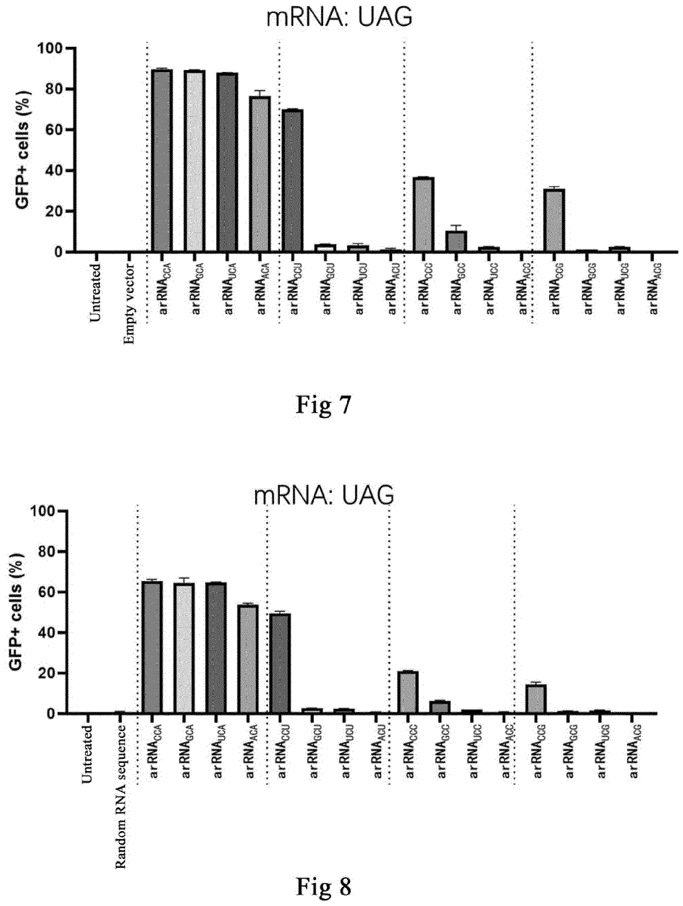

The cells were 293T cells transcribing mRNA with the three-base motif of UAG. After the cells were co-cultured with the arRNA for 72 h (48h after transfection), the FITC channel intensity was analyzed by using the flow cytometer. Results are shown in Fig. 7, UT is a control without any transfection, and Vech is a control that is added with an RNAiMAX transfection reagent and is not transfected with any dRNA.

Then, this experiment was repeated.

Experimental results are shown in Fig. 8. In the first experiment, the arRNA as dry powder was directly dissolved, then storing at -80°C after dissolution. In the repeated experiment, since the arRNA was subjected to freezing and thawing at -80°C once, the overall efficiency was reduced, but the results show a general trend similar to that of the first experiment. Particularly, arRNA_{Ran} is a control transfected with a random RNA sequence.

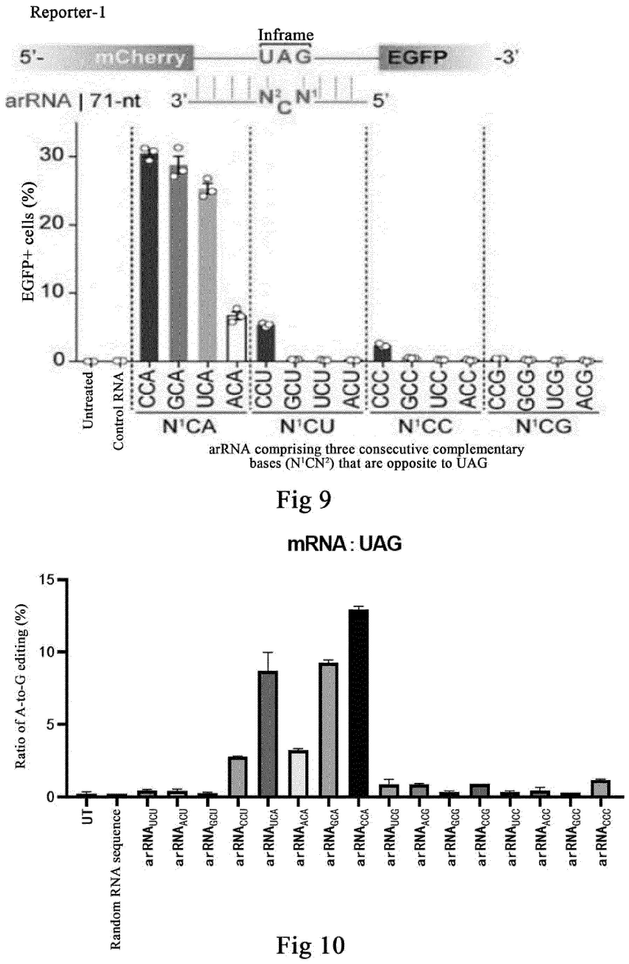

In the prior art, the editing efficiency of the LEAPER system by using a reporter system similar to that in the present example for the three-base motif of UAG is shown in Fig. 9 (Qu et al., 2019). The annotations on the horizontal axis in Fig. 9 are the names of the arRNA sequences, which correspond to subscripts of the names on the horizontal axis in Fig. 7 or Fig. 8. For convenience of comparison, the arRNAs in Fig. 7 or Fig. 8 are arranged in the same order as the arRNAs in Fig. 9. The chemically synthesized arRNAs are used for transfection in the present example, and plasmids are used for transfection in Fig. 9, so the overall editing efficiency of the present example is relatively high but an overall trend is the same as that of Fig. 9. That is, in a case that the three-base motif is UAG, the efficiency of an arRNA is the highest, when the base (corresponding to the target A) in the arRNA is C and the other two bases corresponding to the upstream residue U and the downstream residue G are A and C respectively paired with U and G, i.e., the corresponding arRNA is arRNA_{CCA}. However, in a case that the three-base motif is UAG, the editing efficiency of an arRNA is not improved significantly but reduced, when the bases (respectively corresponding to the upstream residue U and the downstream residue G) in the arRNA are other unpaired bases.

The research results of editing for the three-base motif of UAG according to the present example are substantively the same as those reported in the documents, i.e., when the three-base motif of UAG is edited, the editing efficiency of an arRNA cannot be improved by introducing more unmatched moieties into the three bases (corresponding to the three-base motif) in the arRNA.

### Example 3: RNA editing efficiency for a three-base motif of GAN

In the present example, the reporter system cells respectively comprising three-base motifs of UAG, GAA, GAU, GAC, and GAG were transfected with 16 types of arRNA respectively, and the transfection procedure was the same as that in Example 2.

After 72 h (48h after transfection), samples were collected by using TRIZOL, and RNA (TRIzol Reagent, ambion REF15596026) was extracted. 1 µg of RNA was reversely transcribed with 20 µL of reverse transcription system (TransScript^{®} One-Step gDNA Removal and cDNA Synthesis SuperMix, TransGen AT311-02), and PCR amplification was performed with 1 µL of reverse transcription product by using the following pair of primers: ggagtgagtacggtgtgcGACGAGCTGTACAAGCTGCAGGG (SEQ ID NO: 1), and gagttggatgctggatggTGGTGCAGATGAACTTCAGGGTCAG (SEQ ID NO: 2) (small letters represent primer adapters required by a Hi-Tom kit), and a library was built by using a Hi-Tom kit (Novogene, REF PT045).

Then, next-generation sequencing was performed according to the following procedure, and the A->G editing efficiency for an editing position was analyzed.

### i. Illumina sequencing

The built sequencing library was subjected to high-throughput sequencing by means of PE150 on the NovaSeq6000 platform.

### ii. Processing of sequencing data

Raw data obtained by the high-throughput sequencing were quality-controlled with fastp (v0.19.6) to filter out sequences with low quality, adapter sequences, or polyG. The obtained high-quality sequencing data were split into each sample according to a corresponding barcode sequence with a self-developed split script, comparing with the sequence of the amplified target region by using the BWA (v0.7.17-r1188) software, subjecting to format conversion to generate a BAM file and statistical comparison information by using SAMtools (v1.9), then rearranging, and indexing.

### iii. Analysis of editing efficiency

All potential RNA editing positions were detected by using the JACUSA (v1.3.0) software, and used parameters were call-1-a B,R,D,I,Y,M:4-C ACGT-c2-p1-P UNSTRANDED-R-u DirMult-CE. After high-frequency point mutations present in the controls and the treated samples were filtered out, a value three times the average mutation frequency except for the A->G mutation was taken as a threshold value, and a part of the A->G mutation frequency of an editing position that is greater than the threshold value was taken as the true frequency of the mutation of target A to G.

Experimental results of the three-base motif of UAG are shown in Fig. 10. It can be obviously seen that, the editing efficiency of the arRNA sequence designed according to the principle reported in the prior art is the highest, i.e., arRNA_{CCA} comprising a single mismatch at a position corresponding to a target residue. The results are consistent with the results of the previous GFP experiment.

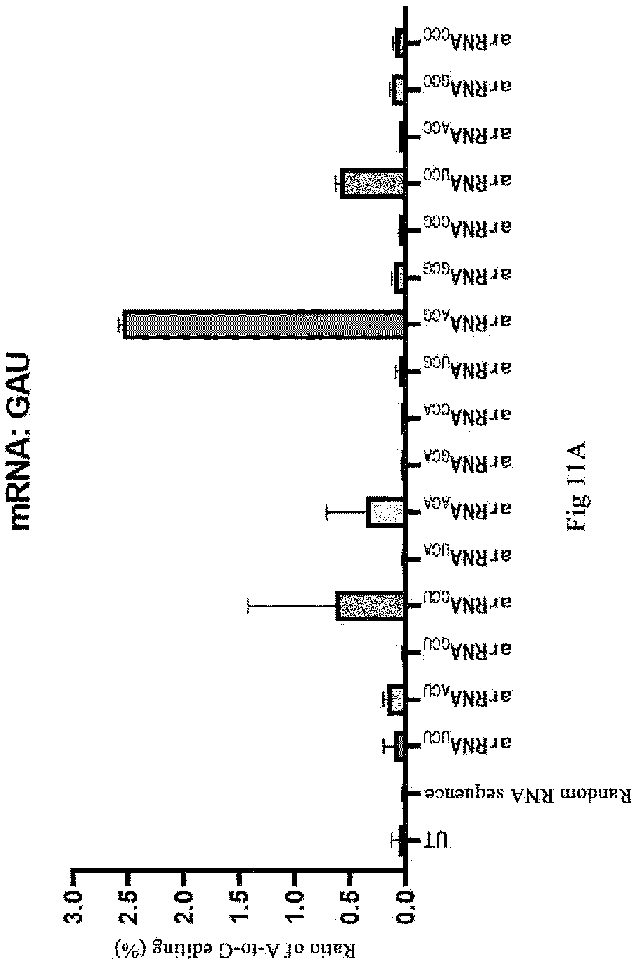

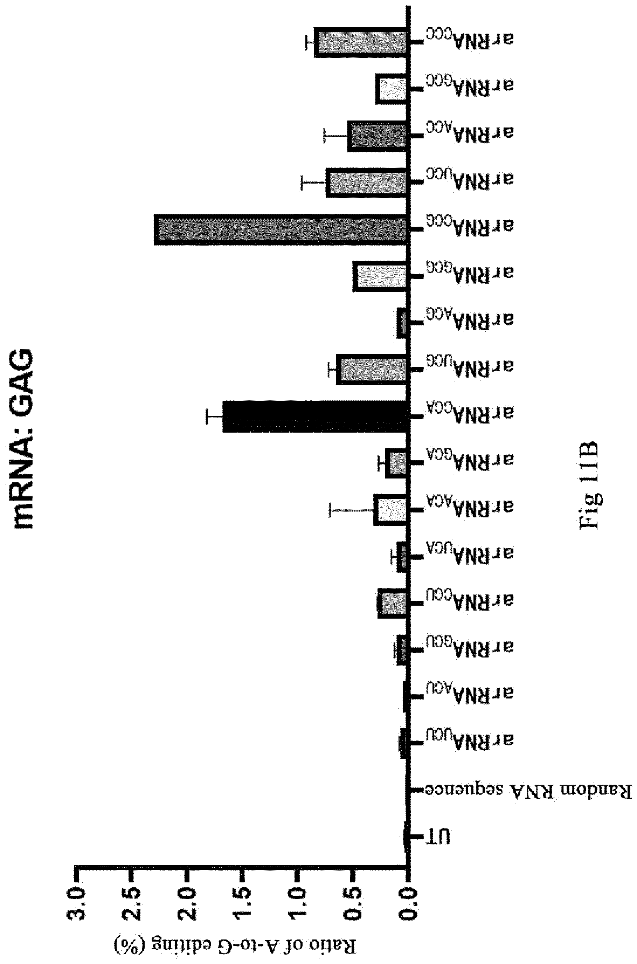

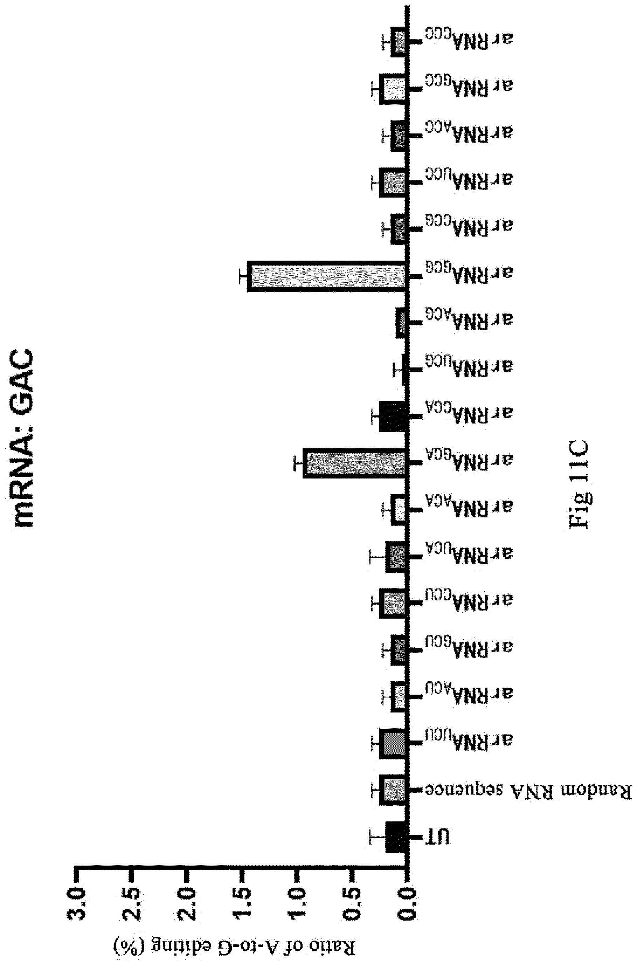

With respect to three-base motifs of GAN (N is any one of four ribonucleotides) for which editing efficiencies of the prior art are very low, the arRNA of the present application achieves unexpected editing effects, as shown in Fig. 11. The efficiency of the arRNA of the present application for GAU is particularly obviously improved. In a case that a three-base motif in an mRNA sequence is GAU, the editing efficiency of arRNA_{ACC} designed by the conventional method in the prior art is substantively 0, which is consistent with the document reports. However, when the complementarity is reduced, even if the base (opposite to the 5'-upstream residue G in the three-base motif) in the arRNA is an unpaired base, i.e., bases other than C, for example, when arRNA_{ACG} is used, the editing efficiency can be greatly improved. As shown in Fig. 11A, the editing efficiency is more than 10 times that of the inherent design (arRNA_{ACC}) in the prior art, and the editing efficiency of arRNA_{ACC} designed according to the prior art is very low, which is consistent with previous reports. In addition, with respect to the three-base motif of GAU, the arRNA designs of arRNA_{ACA}, the arRNAccu, the arRNAucc, etc. of the present example which were appropriately reduced complementarity to the three-base motif have higher editing efficiencies than that of the inherent design (arRNA_{ACC}) in the prior art. It is worthwhile to note that, with respect to the three-base motif of GAU, in a case that the base (opposite to the target A) in the arRNA is C, the base opposite to the downstream residue is a base A complementary to the downstream residue U, and the base opposite to the upstream residue G is a mismatched base G (i.e., arRNA_{ACG}), the editing efficiency is the highest.

Similarly, with respect to the three-base motif of GAC, the editing efficiency of arRNA with appropriately reduced complementarity to the three-base motif is unexpectedly high. As shown in the histogram of Fig. 11C, the editing efficiency of the arRNA_{GCC} designed according to the inherent principle in the prior art is substantively zero, while the editing efficiencies of the arRNAccc and the arRNA_{CCA} with more introduced mismatches are obviously higher. In addition, with respect to the three-base motif of GAC, in a case that the base (opposite to the target A) in the arRNA is C, the base opposite to the downstream residue is a base G complementary to the downstream residue C, and the base opposite to the upstream residue G is a mismatched G (i.e., arRNAccc), the efficiency is the highest.

Similarly, when the three-base motif of GAG is edited (see Fig. 11B), the efficiency of the arRNAccc designed according to the fixed pattern in the prior art is not the highest, while the editing efficiencies of arRNAccc and arRNA_{CCA} with appropriately reduced complementarity are obviously higher. Similar to the above described three-base motifs of GAU and GAC, with respect to the three-base motif of GAG, in a case that the base (opposite to the target A) in the arRNA is C, the base opposite to the downstream residue is a base C complementary to the downstream residue G, and the base opposite to the upstream residue G is a mismatched base G (i.e., arRNAccc), the efficiency is the highest.

Similarly, with respect to GAA, the editing efficiency of the arRNAucc designed according to the fixed pattern in the prior art is not high, while in a case that the base (opposite to the target A) in the arRNA is C, the base opposite to the downstream residue A is a complementary base U, and the base opposite to the upstream residue G is a mismatched base A (i.e., arRNA_{UCA}), the editing efficiency is higher.

In order to further confirm the above results, repeated experiments were performed on mRNAs respectively comprising three-base motifs of GAA, GAU, GAC, and GAG. In the repeated experiments, for each specific three-base motif, only three arRNA designs were repeatedly used.
1. arRNA designed according to the inherent technology, i.e., the base opposite to the target A is C, the other two bases are designed in accordance with the principle of complementary base pairing, and in this case the base paired with the upstream residue G adjacent to the target A is C.
2. According to the design of the present application, the base paired with the upstream residue G adjacent to the target A is A.
3. According to the design of the present application, the base paired with the upstream residue G adjacent to the target A is G.

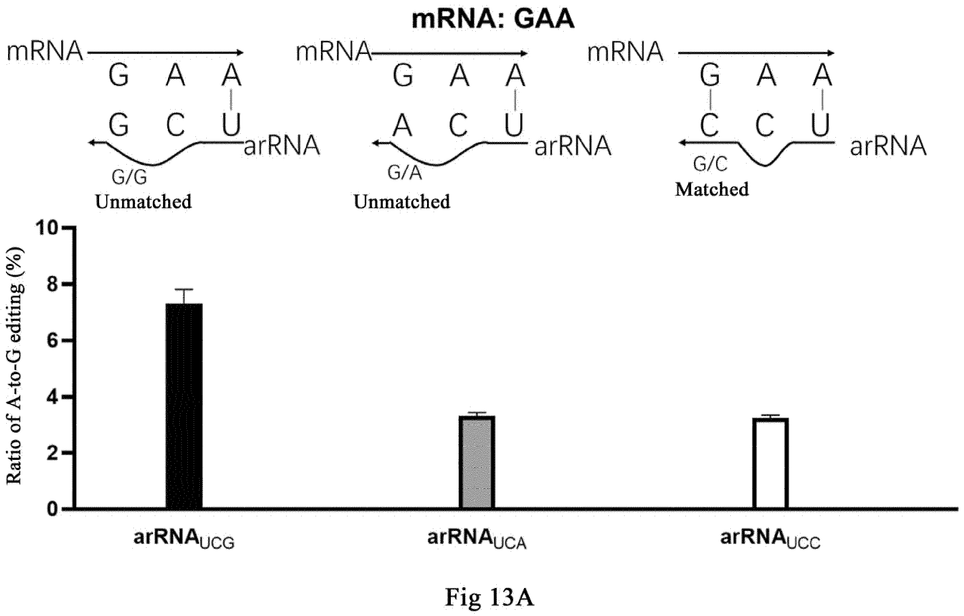

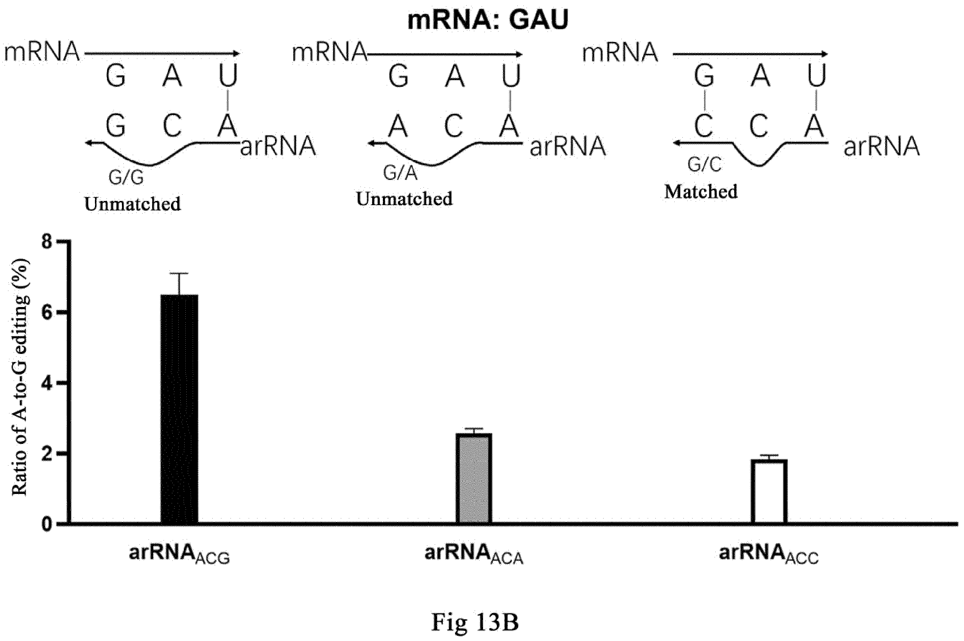

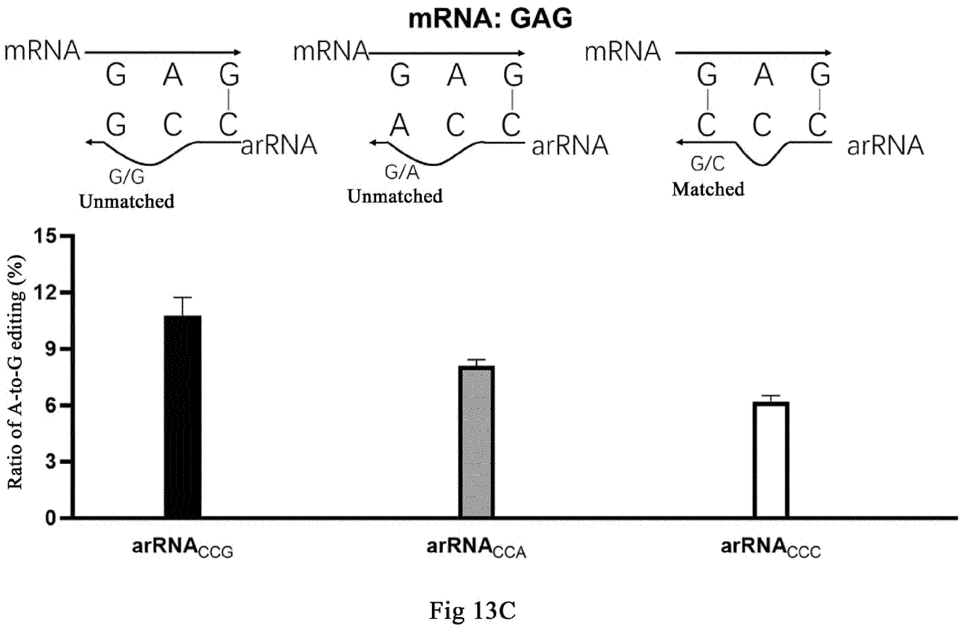

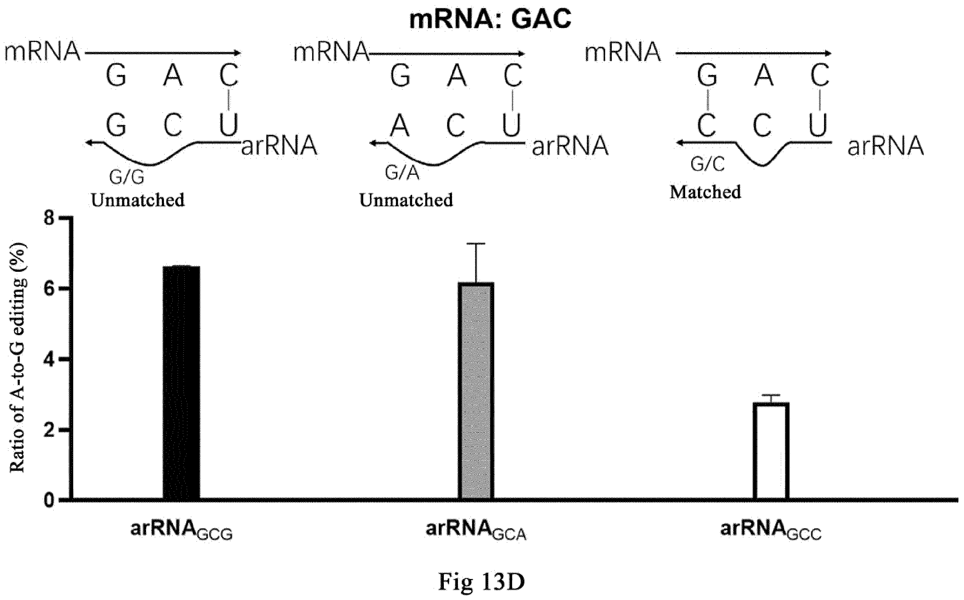

As shown in Fig. 13, no matter the three-base motif is GAA, GAU, GAC or GAG, it can be clearly found that in a case that the base paired with the upstream residue G adjacent to the target residue A is A, the editing efficiency is improved to a certain extent while in a case that the base paired with the upstream residue G adjacent to the target residue A is G, the editing efficiency is usually the highest. In addition, in a case that the base paired with the upstream residue G adjacent to the target residue A is changed from C in the inherent technology to G, the efficiencies for different three-base motifs are improved as follows: GAU>GAC≈GAA>GAG; while the base paired with the upstream residue G adjacent to the target residue A is changed from C in the inherent technology to A, the efficiencies for different three-base motifs are improved as follows: GAC>GAU≈GAG≈GAA.

According to the document reports in the prior art, the efficiencies for the three-base motifs of GAU, GAC and GAA are the lowest, and close to zero (see Fig. 3). Therefore, these three-base motifs should be avoided as much as possible during RNA editing. However, the present application breaks through this limitation by creatively introducing more mismatched bases (opposite to the three-base motif) into the arRNA. It can be seen from the example of the present application that, in a case that among three bases (opposite to the three-base motif) in arRNA, the base opposite to the target A is C, and the base opposite to the upstream and/or downstream residue is a mismatched base, the editing efficiency can be significantly improved. Furthermore, in a case that the upstream residue is G, when the base opposite to the downstream residue is a complementary base, and the base opposite to the upstream residue G is a mismatched base A, the efficiency is higher; particularly, in a case that the base opposite to the upstream residue G is a mismatched base G, the editing efficiency is the highest.

### Example 4: Study on the three-consecutive-base preference for C-to-U RNA editing

### i. Construction of mutant ADAR2-r16-293T

Referring to RESUCE (WO2019071048A9), the ADAR2 catalytic domain was induced to mutate, and the mutation positions were the same as r16 (dADAR2 (E488Q/V351 G/S486A/T37 5S/S370C/P462A/N 597I/L332I/I398V /K350I/M383L/D619G/S582T /V 440I/S495N/K418E/S661T) r16, https://benchling.com/s/seq-19Ytwwh0i0vSIbyXYZ95) in the document. A sequence between an ADAR2 XmaI restriction site and an AscI restriction site in a pLenti-ADAR2 plasmid vector was synthesized in vitro by the conventional DNA synthesis technology (a pLenti-ADAR2 plasmid backbone was donated by Professor Wei Wensheng's laboratory), and the above mutation is comprised in the sequence. Through the two restriction enzymes, a corresponding fragment on the original plasmid pLenti-ADAR2 was replaced with the newly synthesized DNA fragment by restriction enzyme digestion and ligation, and after replacement the plasmid was named as pLenti-ADAR2-r16, and the ADAR2 gene having a mutated catalytic domain according to RESCUE (WO2019071048A9) was named as ADAR2-r16. A full-length cDNA sequence of ADAR2-r16 is shown in Table 6. Through a second-generation lentiviral packaging system (pCAG-VSVG was donated by Arthur Nienhuis & Patrick Salmon (Addgene plasmid #35616; http://n2t.net/addgene:35616; RRID: Addgene_35616); and pCMVR8.74 was donated by Didier Trono (Addgene plasmid # 22036; http://n2t.net/addgene:22036; RRID: Addgene_22036)), pLenti-ADAR2-r16 was packaged as a lentivirus, 293T cells were transfected with the lentivirus, and 48h after transfection, resistance screening was performed with Blasticidin (Solarbio B9300) at a final concentration of 10 µg/mL. After screening, surviving cells were referred to as ADAR2-r16-293T.

### ii. Construction of BFP reporter system

A BFP reporter system was constructed with reference to the document (Vu, L. T., Nguyen, T. T. K., Md Thoufic, A. A., Suzuki, H., & Tsukahara, T. (2016). Chemical RNA editing for genetic restoration: the relationship between the structure and deamination efficiency of carboxyvinyldeoxyuridine oligodeoxynucleotides. Chemical biology & drug design, 87(4), 583-593), and all cDNA sequences of BFP were synthesized in vitro from DNA, the specific sequences are shown in Table 7. The BFP cDNA sequence was cloned into a pCDH-CMV plasmid vector through multiple cloning sites behind the CMV promoter (a pCDH-CMV plasmid skeleton was donated by Kazuhiro Oka, Addgene plasmid #72265; http://n2t.net/addgene:72265; RRID: Addgene_72265). A C-to-U editing position in the reporter system was the base C of position 199 in the BFP sequence, and the bases of positions 199-201 were CAC, which corresponded to the histidine of position 66.

The bases of positions 198-200 in the sequence are CCA in order, which is named as BFP-CCA, and abbreviated as C*. If the base C of position 199 is deaminated and edited to U at the RNA level, the amino acid of position 66 will be changed, and the BFP fluorescent protein is changed from the original blue fluorescence to green fluorescence, so that a signal can be detected by using a fluorescein isothiocyanate (FITC) channel of flow cytometry. After the nucleotide of position 198 is mutated from C to A, T or G, the codon of the amino acid of position 65 is coded by ACC, ACA, ACT or ACG, and they all respectively encodes threonine, so the mutation at this position is a synonymous mutation. Accordingly, when the upstream residue adjacent to the target residue of position 199 in mRNA is a different base, the C-to-U editing efficiencies can be determined and compared at the same time by using the reporter system. A mutation was introduced into the base of position 198 by using a site-directed mutagenesis kit (Q5^{®} Site-Directed Mutagenesis Kit, NEB E0554S), the bases of positions 198-200 were respectively: GCA named as BFP-GCA, and abbreviated as G*; ACA named as BFP-ACA, and abbreviated as A*; and TCA named as BFP-TCA, and abbreviated as T*. When base C of position 199 was mutated to T, and the bases of positions 198-200 were respectively CTA named as BFP-CUA, and abbreviated as CUA. Through a second-generation lentiviral packaging system (under the same conditions as the above lentiviral packaging of ADAR2-r16), the above four constructed plasmids: BFP-GCA, BFP-ACA, BFP-TCA, and BFP-CCA were respectively packaged as a lentivirus, 293T cells or ADAR2-r16-293T cells were transfected with the lentivirus, 48h after transfection, resistance screening was performed with 500 µg/mL Geneticin (Gibco, Catalog number: 10131035) or Blasticidin (Solarbio B9300) at a final concentration of 10 µg/mL, and after screening the surviving cells were respectively named as 293T-GCA, 293T-ACA, 293T-TCA, 293T-CCA, and ADAR2-r16-GCA, ADAR2-r16-ACA, ADAR2-r16-TCA, ADAR2-r16-CCA.

### iii. Design and synthesis of arRNAs

The term "arRNA" in the present example and the term "dRNA" herein have the same meaning, and are interchangeable. In the present example, the base (opposite to a target residue in a three-base motif) in arRNA was located in the middle of the arRNA, and the 5' upstream and the 3'-downstream extend to both sides by the same length. Due to the limitation of synthesized length, in the present example, RNA with a length of 91 nt was first synthesized in vitro, and according to different nucleotides position 46 (targeting base), i.e., the nucleotide of position 46 is A, U, G or C respectively, the four synthesized arRNAs were respectively abbreviated as A*, U*, G*, and C*. Specific sequences of the four synthesized arRNAs are shown in Table 5. The difference from the design method of the LEAPER technology (WO2020074001A1) lies in that: in the four arRNAs designed in this experiment, only the targeting base opposite to the target residue C was changed, i.e., the base of position 46 was A, U, G or C, and the base of position 47 (the position 198 in the corresponding reporter system) in the arRNA was designed according to the BFP sequence before introduction of a mutation, i.e., CCA. Then, arRNAs respectively comprising different three consecutive complementary bases were synthesized under the conditions that the target residue in the three-base motif was cytidine and the 'upstream residue was adenosine, and specific sequences are shown in Table 8. The nucleotide of position 46 in the arRNA was fixed as U, and nucleotides of positions 45 and 47 were respectively A, U, G or C, and a total of 16 arRNAs were synthesized. Each arRNA was named according to the following principles: all arRNAs were named by starting with "arRNA", and then three consecutive complementary bases in arRNA were displayed in the form of subscripts. On the basis of the target residue C in mRNA corresponds to a targeting base of U in arRNA, three consecutive complementary bases were displayed in the order of the 5'-end to the 3'-end. For example, arRNA relative to the three-base motif of CCA, the upstream residue adjacent to the target residue C is C, and the corresponding 3' nearest-neighbor residue of targeting base in arRNA is G; for the targeting base C, the corresponding targeting residue in the arRNA is U; the downstream residue adjacent to the target residue C is A, and the corresponding 5' nearest-neighbor residue of the targeting base in the arRNA is U, thus the three consecutive complementary bases in the arRNA are UUG, and according to the naming rules, this antisense RNA is named as arRNA_{UUG}. In order to unify the nomenclature of the first batch of four synthesized arRNAs (A*, U*, G*, and C*) and the second batch of 16 synthesized RNAs, the first batch of four synthesized arRNAs (A*, U*, G*, and C*) were respectively named as arRNA_{UAG}, arRNA_{UUG}, arRNA_{UGG}, and arRNAucc in the following experiment. It should be noted that in the two experiments, a sequence of the arRNA_{UUG} synthesized for the first time is exactly the same as that of the arRNA_{UUG} synthesized for the second time, and the arRNA_{UUG} was synthesized in two different batches.

### iv. Target C para-antisense RNA test

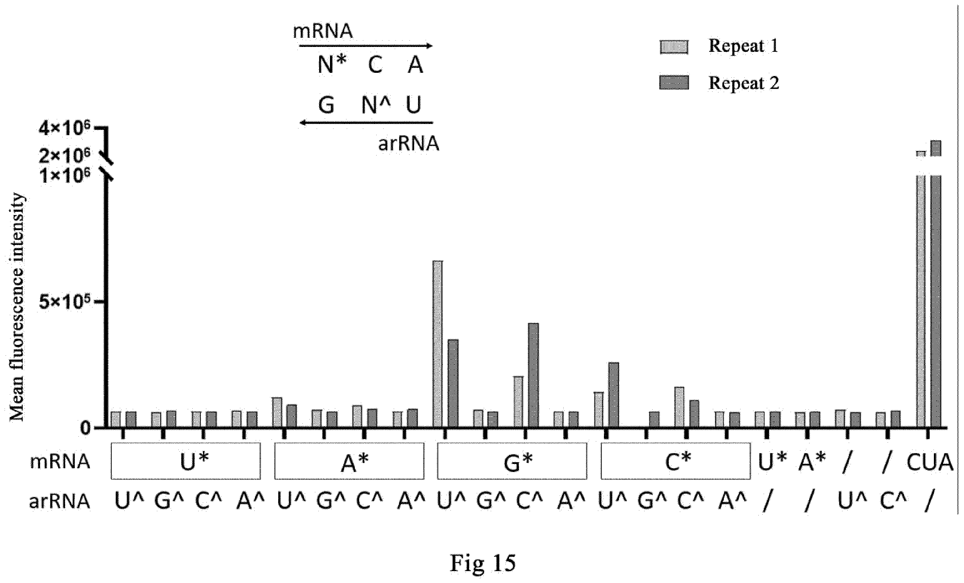

ADAR2-r16-293T cells were plated onto a 6-well plate at a density of 300000 cells/well, 24 h after plating, the cells were transfected with Lipofectamine^{™} 3000 Transfection Reagent (Invitrogen, Catalog number: L3000015), the transfection procedure was performed according to the instructions. Two repeated experiments were performed with Lipofectamine 3000 transfection reagents at different concentrations according to the instructions, and 3.75 µL and 7.5 µL transfection reagent concentration per well were respectively used in Repeat 1 and Repeat 2. 2.5 µg of BFP and relevant plasmid, i.e., BFP-GCA (abbreviated G*), BFP-ACA (abbreviated A*), BFP-TCA (abbreviated T*), or BFP-CUA (abbreviated CUA), was added, 25 pmol of synthesized guide RNA was added, 48h after transfection, the FITC channel signal intensity was detected by FACS. Statistical results of mean fluorescent intensity (MFI) for positive cells are shown in Fig. 15.

In Fig. 15, the mRNA row indicates the BFP reporter system plasmid added in the corresponding well, and the arRNA row indicates the arRNA added in the corresponding well. In the BFP reporter system, the bases of positions 198-200 in the original sequence are CCA, when the base C of position 198 is converted to A or T or G, the amino acid of position 65 is converted to threonine, so changes of the base of position 198 in the four different reporter systems of BFP-GCA, BFP-CCA, BFP-ACA, and BFP-TCA will not affect the original protein function. As shown in Fig. 15, in a case that no arRNA is added, MFI of the background GFP signal of the reporter system is 5×10⁴ (the reporter system is marked as U*, arRNA is marked as /; and the reporter is marked as A*, and arRNA is marked as /). However, after the base C of position 199 is mutated to T by a point mutation at the DNA level (the three-base motif in mRNA is CUA), MFI of a GFP signal is about 2.4×10⁶-3.1×10⁶, which is about 100 times that of the background signal. Therefore, it indicates that if the base C of position 199 is converted to U at the RNA level, MFI of a GFP signal will be increased by about 100 times.

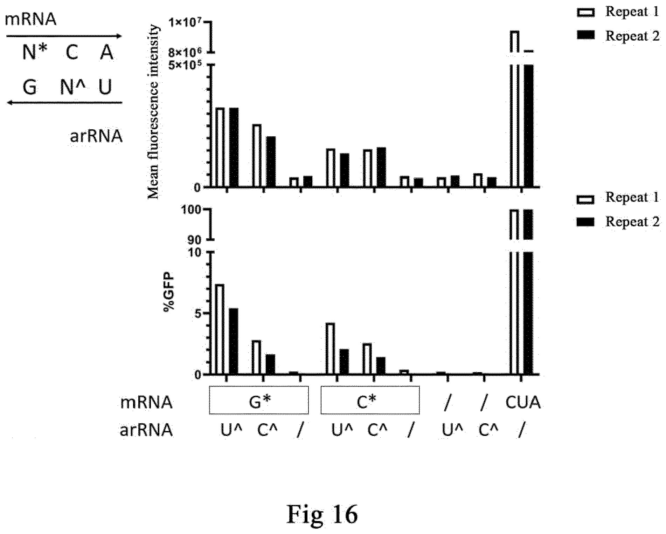

However, after arRNA is added, on the basis of unchanged base C of position 199 at the DNA level, as shown in Fig. 15, MFI of a final GFP signal can be increased to greater than 5×10⁵, and the fluorescence intensity is about 20% of the fluorescence intensity in a case that the base C of position 199 is mutated to T. In order to further confirm the editing capability and the base preference, the above experiment was further designed and repeated. Results are shown in Fig. 16, the experimental conditions are substantively the same as those in Fig. 15, and the only difference is that: 3.75 µL of transfection reagent is used in Repeat 1 and Repeat 2. That is, in a case that the three-base motif is GCA or CCA, the efficiency of arRNA of U^ (arRNA_{UUG}) is higher than that of arRNA of C^ (arRNA_{UCG}). Compared to Fig. 15, MFI are decreased by nearly half under the same experimental conditions in Fig. 16, which is due to the fact that the arRNA in Fig. 15 was tested immediately after the dry powder of arRNA was dissolved, while the experiment in Fig. 16 was performed after the arRNA solution in the experiment of Fig. 15 was frozen and thawed once at -80°C. However, it can be seen that although the maximum value is decreased compared to Fig. 15, the four highest editing efficiency results in Fig. 15 are substantively repeated in the experiment in Fig. 16, and the efficiency in Fig. 16 shows the same trend. Under the test design conditions of the present example, the 5'-end base in the three consecutive complementary bases in arRNA is fixed to U, and the 3'-end base is fixed to G, the study focuses on the base (opposite to the target C) in the three consecutive complementary bases, and the following conclusions are put forward: the editing efficiency of the three consecutive complementary bases having the middle residue of U^ is higher than that of the three consecutive complementary having the middle residue of C^, and the applicant has found that when the upstream residue in the three-base motif is changed, the maximum efficiency for the three-base motif of GCA is higher than that for CCA.

### v. Three-consecutive-base preference test

For better consistency of the subsequent results, according to the description of ii. Construction of BFP reporter system, the applicant integrated the four plasmids: BFP-GCA, BFP-ACA, BFP-TCA, and BFP-CCA respectively into ordinary 293T cells without ADAR2-r16 by lentiviral packaging and 293T cells stably integrated with ADAR2-r16, and the procedure and nomenclature referred to ii. Construction of BFP reporter system. Because the reporter system was integrated into the cell genome, different transfection reagents were used in the transfection with arRNA in the three-consecutive-base preference test, and the transfection with arRNA in the target C para-antisense RNA test. In the target C para-antisense RNA test, it is necessary to transfect with arRNA and plasmids at the same time, so as described above, Lipofectamine 3000 was used. In the three-consecutive-base preference test, it is only necessary to transfect with arRNA, and the plasmids is not used, so Lipofectamine^{™} RNAiMAX Transfection Reagent (Invitrogen, Catalog number: 13778100) was used. 293T cells or ADAR2-r16-293T cells comprising different reporter systems were plated onto a 12-well plate at a density of 150000 cells/well, 24 h after plating, the cells in each well were transfected with 15 pmol of arRNA by using an RNAiMAX reagent, 48h after transfection, the FITC channel signal intensity was detected by FACS, and the percentage of GFP+ cells was counted.

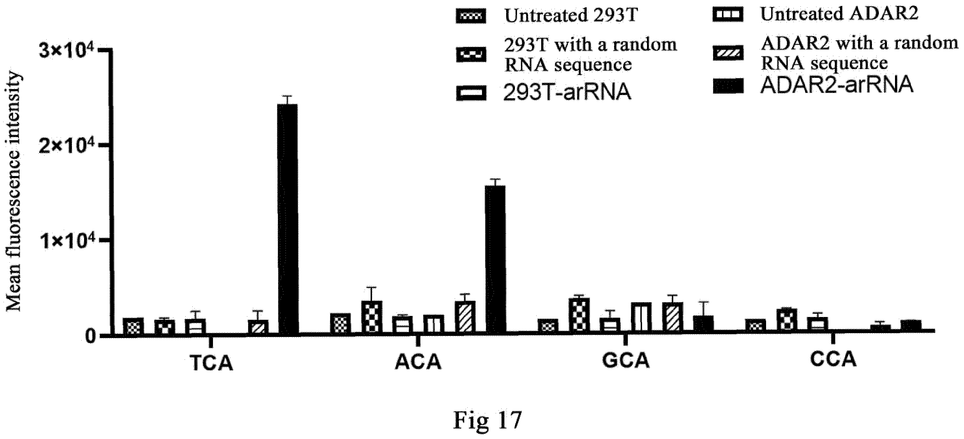

As for the case that the three consecutive complementary bases in arRNA comprise a single mismatch with the target C, and the mismatched base corresponding to the target C is U, and the other two bases are respectively completely matched with the upstream residue and downstream residue adjacent to the target C (i.e., in a case that the reporter system is BFP-GCA, the three consecutive complementary bases (complementary to the reporter system) in the arRNA is UUC; in a case that the reporter system is BFP-ACA, the three consecutive complementary bases (complementary to the reporter system) in the arRNA is UUU; in a case that the reporter system is BFP-TCA, the three consecutive complementary bases (complementary to the reporter system) in the arRNA is UUA; and in a case that the reporter system is BFP-CCA, the three consecutive complementary bases (complementary to the reporter system) in the arRNA is UUG), the test results are shown in Fig. 17. In this figure, "untreated" indicates a control without arRNA, "random RNA sequence" indicates a control added with a random RNA sequence of 91 nt ( the specific sequence is shown as Ran-91 in Table 8), and "arRNA" indicates addition of the corresponding matched arRNA according to the above rules. It can be seen from Fig. 17, in a case that the three consecutive bases are TCA or ACA, the editing efficiency for the system is high, and in a case that the three consecutive bases are GCA or CCA, the editing efficiency is close to zero.

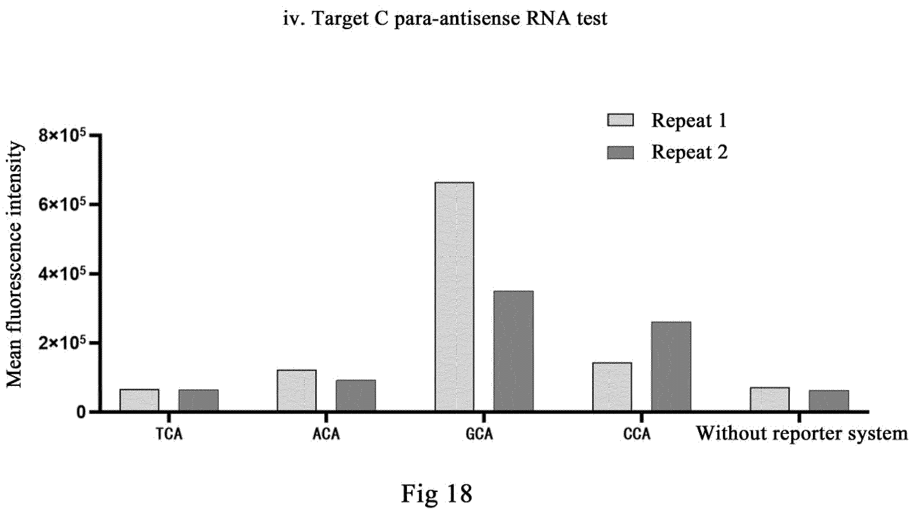

The results of the three-consecutive-base test once brought great troubles to this study. In the test in Fig. 15, the three consecutive bases comprise a base A, U, C or G corresponding to the target C, while in the test in Fig. 17, C is paired with U. The data of the case where the base (corresponding to the target C) in the arRNA is U in the experiment corresponding to Fig. 15 was separately extracted, and redrawn to obtain Fig. 18. By comparison with Fig. 17, it can be found that the conclusions of the two experiments are obviously contradictory. Although the statistical patterns in the two figures are different, the trends in the same batch of experiments are significantly different. In Fig. 18 redrawn according to the data in Fig. 15, efficiencies for GCA and CCA are high, while in Fig. 17, efficiencies for TCA and ACA are obviously high.

### vi. Unexpected finding of the case of mismatching with the 5'-upstream adjacent to an editing position

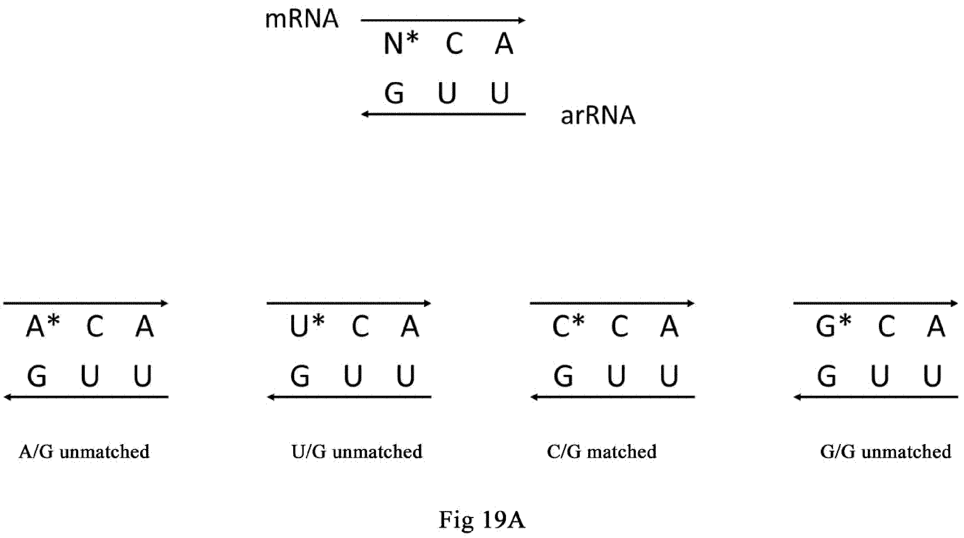

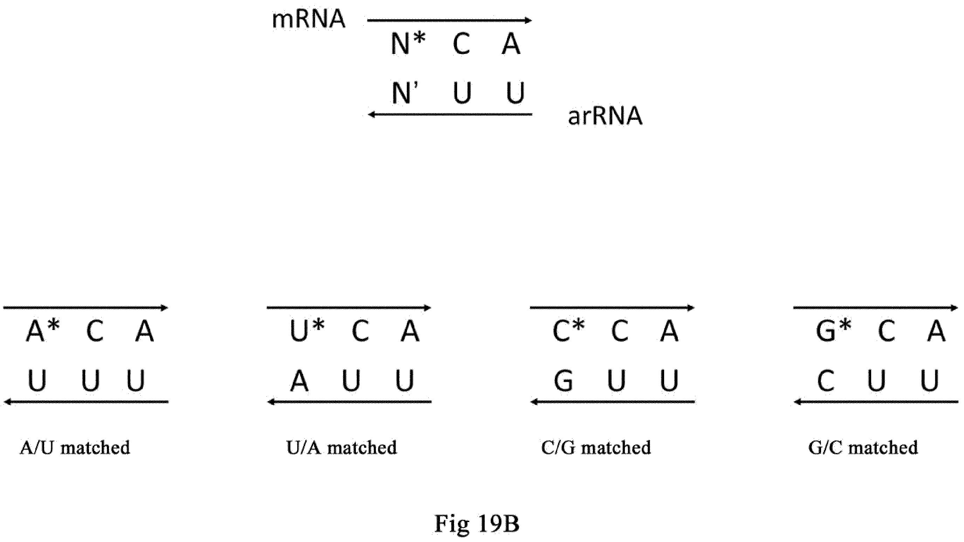

The contradictory results of the two experiments are completely unexpected. By multiple repeated experiments and two careful comparisons of the arRNAs, subtle differences in the RNA design in the two experiments were unexpectedly discovered and replicated. Fig. 19A shows the pairing relationship between the three-base motif in mRNA used in Fig. 18 and the three consecutive complementary bases in the arRNA, and Fig. 19B shows the pairing relationship between the three-base motif in mRNA used in Fig. 17 and the three consecutive complementary bases in the arRNA. By comparison, it can be found that the difference between the two is that: the former (Fig. 19A) shows the case that the base (opposite to the upstream residue adjacent to the target C) in the arRNA is G, and the upstream residue is mismatched with the opposite base in the arRNA except for the case that the upstream residue adjacent to the target C is C; and the latter (Fig. 19B) shows the case that the base (opposite to the upstream residue adjacent to the target C) in the arRNA is a base strictly complementary to the upstream residue. Therefore, the inventors speculated that the reason for the above contradiction was that a mismatch between the base in the three consecutive complementary bases in the arRNA and the upstream residue may lead to a change in the three-consecutive-base preference.

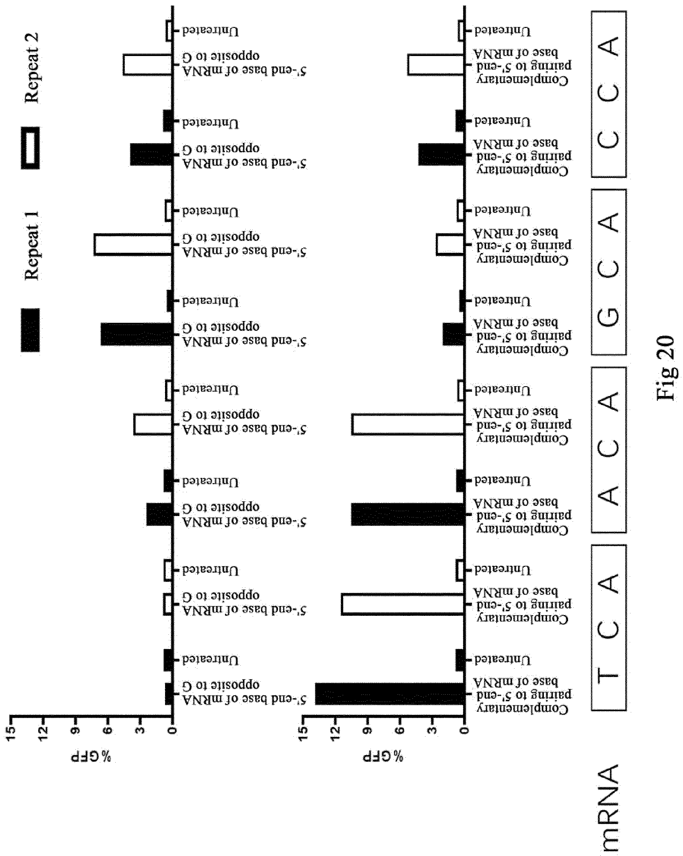

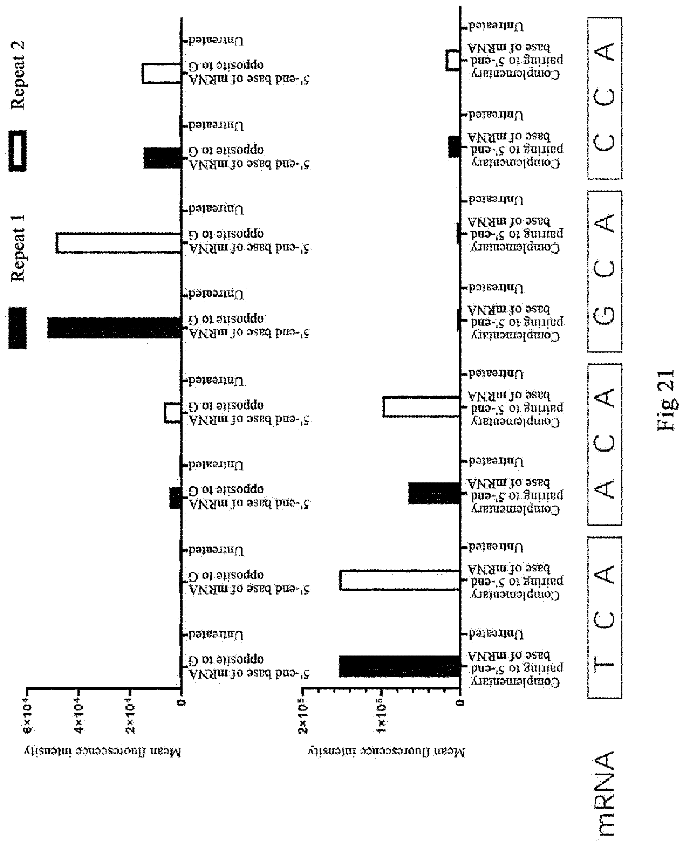

In order to further verify the above speculation, the arRNAs synthesized in iv. Target C para-antisense RNA test, and the arRNAs synthesized in v. Three-consecutive-base preference test were tested together, and GFP percentages and MFI were counted. The tests conditions were exactly the same as those in v. Three-consecutive-base preference test. Particularly, the upper panels of Fig. 20 and Fig. 21 show test results of the arRNAs synthesized in iv. Target C para-antisense RNA test, and the lower panels of Fig. 20 and Fig. 21 show test results of the arRNAs synthesized in v. Three-consecutive-base preference test. The addition of corresponding arRNAs were the same as that in the two tests of iv and v. As shown in Fig. 20 (%GFP) and Fig. 21 (MFI), Repeat 1 and Repeat 2 are two independent experiments. It can be seen from Fig. 20 and Fig. 21 that, although the statistical patterns in the two figures are different, the trends in the two figures are similar. In the upper panels, the editing efficiencies for GCA and CCA are relatively high, while the editing efficiencies for TCA and ACA are relatively low, which is consistent with the conclusion in iv. Target C para-antisense RNA test. In the lower panels, the editing efficiencies for TCA and ACA are relatively high, while the editing efficiencies for GCA and CCA are close to zero, which is consistent with the conclusion in v. Three-consecutive-base preference test. Therefore, the above speculation was confirmed, i.e., the two seemingly contradictory conclusions were caused by different arRNA design methods. The inventors have also unexpectedly found that with respect to the three-base motif of GCA, the editing efficiency of the arRNA designed according to the prior art is close to zero, and if an additional G-G mismatch is added, the editing efficiency is significantly improved.

Further inspired by the above findings, the inventors considered whether the editing efficiency can be further improved by introducing other additional unmatched sequences into three-base motifs. With this inspiration, on the premise that the base (opposite to the target C) in the arRNA was U, more mutations were introduced to the positions (opposite to the upstream residue and/or the downstream residue adjacent to the target base in the three-base motif) in the three consecutive complementary bases in the arRNA. Since the base opposite to the upstream residue of the target base may be A, U, C or G, and the other base opposite to the downstream residue of the target base may also be A, U, C or G, there are a total of 16 three-consecutive-complementary-base sequences: AUA, AUU, AUC, AUG, UUA, UUU, UUC, UUG, CUA, CUU, CUC, CUG, GUA, GUU, GUC, and GUG. In view of this, arRNAs comprising the above 16 three-consecutive-complementary-base sequences were synthesized, and they were named according to the three-consecutive-complementary-base sequences, the specific sequences are shown in Table 8. These 16 different arRNAs were respectively transfected into 8 previously constructed cell lines comprising reporters by RNAiMAX, i.e., BFP-ACA-293T and BFP-ACA-293T-ADAR2-r16 (see Fig. 22B), BFP-TCA-293T and BFP-TCA-293T-ADAR2-r16 (see Fig. 22A), BFP-CCA-293T and BFP-CCA-293T-ADAR2-r16 (see Fig. 22D), and BFP-GCA-293T and BFP-GCA-293T-ADAR2-r16 (Fig. 22C), and the transfection conditions and test time were the same as those in the experiments related to Fig. 17. The controls in the 4 Figs 22A-D are the same sample, "random sequence of 91 nt" is a control added with a random sequence of 91 nt, "vector only" is a control added with an RNAiMAX transfection reagent but not added with RNA, "Opti-DMEM medium" is a control added with an equal volume of Opti-DMEM but not added with an RNAiMAX transfection reagent, "untreated" is a control without transfection, particularly, arRNA_{UAG}, arRNA_{UUG}, arRNA_{UCG}, and arRNAucc respectively comprise the same sequence as CCA-arRNA_{UAG}, CCA-arRNA_{UUG}, CCA-arRNAucc, and CCA-arRNAucc, but they are synthesized in two different batches.

Fig. 22 shows the preferred selections for introducing more mismatches, i.e., in a case that the three-base motif is ACA, the editing efficiency of arRNA having the three consecutive complementary bases of AUU or GUU is high, particularly the editing efficiency of arRNA having the three consecutive complementary bases of AUU is higher; in a case that the three-base motif is UCA (TCA in the plasmid), the editing efficiency of arRNA having the three consecutive complementary bases of AUA, GUA or CUA is high, particularly the editing efficiency of arRNA having the three consecutive complementary bases of AUA is higher; in a case that the three-base motif is GCA, the editing efficiency of arRNA having the three consecutive complementary bases of UUG or UCG is high, particularly the editing efficiency of arRNA having the three consecutive complementary bases of UUG is higher; and in a case that the three-base motif is CCA, the editing efficiency of arRNA having the three consecutive complementary bases of AUG is higher.

In addition, different upstream residues in the target RNA may lead to different editing efficiencies. In order to better define the scope of application of the present application and the preferred order of the three-base motifs, in the present example the editing efficiency in the case of mismatches directly opposite to the upstream residue and/or the downstream residue and the editing efficiency in the case of a single mismatch opposite to the target residue were compared. The results are also shown in Fig. 22, it can be found that in a case that the upstream residue in the three-base motif is A or U, the arRNA having mismatches directly opposite to the upstream residue and/or the downstream residue can reach an editing efficiency comparable to that of the arRNA with a single mismatch opposite to the target residue. For example, in a case that the three-base motif is ACA, the arRNA comprising the three-consecutive-complementary-base sequence UUU having a single base mismatch opposite to the target residue can reach an editing efficiency comparable to that of AUU or GUU having mismatches directly opposite to the upstream residue and/or the downstream residue; and in a case that the three-base motif is UCA, the arRNA comprising the three-consecutive-complementary-base sequence UUA having a single mismatch opposite to the target residue can reach an editing efficiency comparable to that of AUA having mismatches directly opposite to the upstream residue and/or the downstream residue. However, in a case that the three-base motif is GCA, the editing efficiency of the three-consecutive-complementary-base sequence UUC having a single base mismatch opposite to the target residue is close to 0, and the editing efficiency of UUG or UCG having mismatches directly opposite to the upstream residue and/or the downstream residue may be several times to more than 10 times that of UUC. In a case that the three-base motif is CCA, the editing efficiency of AUG introducing mismatches directly opposite to the upstream residue and/or the downstream residue is also similar to that of UCG. Accordingly, it can be seen that according to the order of the improvements of the editing efficiency, the preferred order of the mismatches of the upstream residue in the three-base motif is as follows: G>C>A≈U, i.e., in a case that the upstream residue in the three-base motif is G, the editing efficiency can be significantly improved by introducing G that is mismatched with the upstream residue.

Finally, it is worthwhile to note that because the data in Fig. 22 are obtained from the same batch of tests under the same experimental conditions by the same detection method, it is convenient to compare the editing efficiencies of the C-to-U RNA editing technology for the four different motifs of ACA, UCA, CCA, and GCA. As shown in Fig. 22, among the three-base motifs, the maximum efficiency for ACA or UCA is about 10% GFP+; with respect to GCA, the editing efficiency of the arRNA having a single mismatch opposite to the target base is close to 0; and the editing efficiency of the arRNA that not only comprises a mismatch opposite to the target base but also is introduced with mismatches directly opposite to the upstream residue and/or the downstream residue can be increased to 6%-8% GFP+; however, with respect to CCA, the maximum efficiency of arRNA that is introduced with mismatches directly opposite to the upstream residue and/or the downstream residue is not greater than 2.5% GFP+.

### Industrial Applicability

The present application breaks through the limitation of the low editing efficiency of the existing RNA editing technology for three-base motifs of GAU, GAC, etc., so that the three-base motifs starting with G can still be edited with considerable efficiency to break through the embarrassing situation that the existing RNA editing technology cannot edit the sites of GAU, GAC, etc., thereby significantly improving the editing efficiencies of the ADAR-based RNA editing systems (e.g., LEAPR (WO2020074001A1) and RESTORE (WO2020001793A1)) in the prior art for the three-base motifs (other than UAG) that do not meet the natural preference of ADAR. Meanwhile, the technical solutions of the present application also break through the limitation of the low editing efficiency of the existing RNA editing technology for the three-base motifs such as GCA, and compared with the low editing efficiency of RESCUE in the prior art (WO2019071048A9) for the three-base motif of GCA, the present application greatly enhances the ability to edit GCA by introducing additional base mismatches. The present application breaks through the long-lasting limitation in selection of editing sites in the application of RNA editing. For example, in terms of disease therapy development, according to the present application, more inherited diseases caused by gene mutations have the opportunity to be more safely and efficiently treated by RNA editing.

### Sequence Listing

**Table 1: Primers for constructing 16 three-base motif reporter systems**

| Primer | SEQ ID NO | Primer sequence |
|---|---|---|
| Vector-F | 3 | Ctgttttgacctccatagaagacaccgactctagacgtggaacagtacgaacgcgc |
| GAT-R | 4 | Cactggcagagccctatcgcatcgcgagcaggcgct |
| GAT-F | 5 | Tgctcgcgatgcgatagggctctgccagtgagc |
| Vector-R | 6 | gggtttaaacccctgcagggtgtacaccggcgcgccttacttgtacagctcgtccatgc |
| GAA-R | 7 | Cactggcagagccctttcgcatcgcgagcaggcgct |
| GAA-F | 8 | Tgctcgcgatgcgaaagggctctgccagtgagc |
| GAG-R | 9 | Cactggcagagccctctcgcatcgcgagcaggcgct |
| GAG-F | 10 | Tgctcgcgatgcgagagggctctgccagtgagc |
| GAC-R | 11 | Cactggcagagccctgtcgcatcgcgagcaggcgct |
| GAC-F | 12 | Tgctcgcgatgcgacagggctctgccagtgagc |
| AAA-R | 13 | Cactggcagagcccttttgcatcgcgagcaggcgct |
| AAA-F | 14 | Tgctcgcgatgcaaaagggctctgccagtgagc |
| AAT-R | 15 | cactggcagagccctattgcatcgcgagcaggcgct |
| AAT-F | 16 | tgctcgcgatgcaatagggctctgccagtgagc |
| AAC-R | 17 | cactggcagagccctgttgcatcgcgagcaggcgct |
| AAC-F | 18 | tgctcgcgatgcaacagggctctgccagtgagc |
| AAG-R | 19 | cactggcagagccctcttgcatcgcgagcaggcgct |
| AAG-F | 20 | tgctcgcgatgcaagagggctctgccagtgagc |
| CAA-R | 21 | cactggcagagccctttggcatcgcgagcaggcgct |
| CAA-F | 22 | tgctcgcgatgccaaagggctctgccagtgagc |
| CAT-R | 23 | cactggcagagccctatggcatcgcgagcaggcgct |
| CAT-F | 24 | tgctcgcgatgccatagggctctgccagtgagc |
| CAC-R | 25 | cactggcagagccctgtggcatcgcgagcaggcgct |
| CAC-F | 26 | tgctcgcgatgccacagggctctgccagtgagc |
| CAG-R | 27 | cactggcagagccctctggcatcgcgagcaggcgct |
| CAG-F | 28 | tgctcgcgatgccagagggctctgccagtgagc |
| TAA-R | 29 | cactggcagagccctttagcatcgcgagcaggcgct |
| TAA-F | 30 | tgctcgcgatgctaaagggctctgccagtgagc |
| TAG-R | 31 | cactggcagagccctctagcatcgcgagcaggcgct |
| TAG-F | 32 | tgctcgcgatgctagagggctctgccagtgagc |
| TAC-R | 33 | cactggcagagccctgtagcatcgcgagcaggcgct |
| TAC-F | 34 | tgctcgcgatgctacagggctctgccagtgagc |
| TAT-R | 35 | cactggcagagccctatagcatcgcgagcaggcgct |
| TAT-F | 36 | tgctcgcgatgctatagggctctgccagtgagc |

**Table 2: Materials for constructing 16 three-base motif reporter systems and the assembly sequence thereof**

| Fragment | Source | Plasmid /template | Enzyme/primer | | Correct band | Assembly | Three -base motif |
|---|---|---|---|---|---|---|---|
| 0 | Digestion | Reporter 1 | XbaI | AscI | 7951 | 0 | GAU |
| 1 | PCR | Reporter 1 | Vector-F | GAT-R | 153 | 1 | |
| 2 | PCR | Reporter 1 | GAT-F | Vector-R | 735 | 2 | |
| 3 | PCR | Reporter 1 | Vector-F | GAA-R | 153 | 0 | GAA |
| 4 | PCR | Reporter 1 | GAA-F | Vector-R | 735 | 3 | |
| | | | | | | 4 | |
| 5 | PCR | Reporter 1 | Vector-F | GAG-R | 153 | 0 | GAG |
| 6 | PCR | Reporter 1 | GAG-F | Vector-R | 735 | 5 | |
| | | | | | | 6 | |
| 7 | PCR | Reporter 1 | Vector-F | GAC-R | 153 | 0 | GAC |
| 8 | PCR | Reporter 1 | GAC-F | Vector-R | 735 | 7 | |
| | | | | | | 8 | |
| 9 | PCR | Reporter 1 | Vector-F | AAA-R | 153 | 0 | AAA |
| 10 | PCR | Reporter 1 | AAA-F | Vector-R | 735 | 9 | |
| | | | | | | 10 | |
| 11 | PCR | Reporter 1 | Vector-F | AAT-R | 153 | 0 | AAU |
| 12 | PCR | Reporter 1 | AAT-F | Vector-R | 735 | 11 | |
| | | | | | | 12 | |
| 13 | PCR | Reporter 1 | Vector-F | AAC-R | 153 | 0 | AAC |
| 14 | PCR | Reporter 1 | AAC-F | Vector-R | 735 | 13 | |
| | | | | | | 14 | |
| 15 | PCR | Reporter 1 | Vector-F | AAG-R | 153 | 0 | AAG |
| 16 | PCR | Reporter 1 | AAG-F | Vector-R | 735 | 15 | |
| | | | | | | 16 | |
| 17 | PCR | Reporter 1 | Vector-F | CAA-R | 153 | 0 | CAA |
| 18 | PCR | Reporter 1 | CAA-F | Vector-R | 735 | 17 | |
| | | | | | | 18 | |
| 19 | PCR | Reporter 1 | Vector-F | CAT-R | 153 | 0 | CAU |
| 20 | PCR | Reporter 1 | CAT-F | Vector-R | 735 | 19 | |
| | | | | | | 20 | |
| 21 | PCR | Reporter 1 | Vector-F | CAC-R | 153 | 0 | CAC |
| 22 | PCR | Reporter 1 | CAC-F | Vector-R | 735 | 21 | |
| | | | | | | 22 | |
| 23 | PCR | Reporter 1 | Vector-F | CAG-R | 153 | 0 | CAG |
| 24 | PCR | Reporter 1 | CAG-F | Vector-R | 735 | 23 | |
| | | | | | | 24 | |
| 25 | PCR | Reporter 1 | Vector-F | TAA-R | 153 | 0 | UAA |
| 26 | PCR | Reporter 1 | TAA-F | Vector-R | 735 | 25 | |
| | | | | | | 26 | |
| 27 | PCR | Reporter 1 | Vector-F | TAG-R | 153 | 0 | UAG |
| 28 | PCR | Reporter 1 | TAG-F | Vector-R | 735 | 27 | |
| | | | | | | 28 | |
| 29 | PCR | Reporter 1 | Vector-F | TAC-R | 153 | 0 | UAC |
| 30 | PCR | Reporter 1 | TAC-F | Vector-R | 735 | 29 | |
| | | | | | | 30 | |
| 31 | PCR | Reporter 1 | Vector-F | TAT-R | 153 | 0 | UAU |
| 32 | PCR | Reporter 1 | TAT-F | Vector-R | 735 | 31 | |
| | | | | | | 32 | |

**Table 3: arRNA sequences used in Examples 1 to 3**

| arRNA | SEQ ID NO | Sequence (5'-3') |
|---|---|---|
| arRNAucu | 37 | mG*mA*mU*GGGCACCACCCCGGUGAACAGCUCCUCG CCCUUGCUCACUGGCAGAGCCCUUCUGCAUCGCGAGC AGGCGCUGCCmU*mC*mC |
| arRNA_{ACU} | 38 | mG*mA*mU*GGGCACCACCCCGGUGAACAGCUCCUCG CCCUUGCUCACUGGCAGAGCCCUACUGCAUCGCGAGC AGGCGCUGCCmU*mC*mC |
| arRNAccu | 39 | mG*mA*mU*GGGCACCACCCCGGUGAACAGCUCCUCG CCCUUGCUCACUGGCAGAGCCCUGCUGCAUCGCGAGC AGGCGCUGCCmU*mC*mC |
| arRNA_{CCU} | 40 | mG*mA*mU*GGGCACCACCCCGGUGAACAGCUCCUCG CCCUUGCUCACUGGCAGAGCCCUCCUGCAUCGCGAGC AGGCGCUGCCmU*mC*mC |
| arRNA_{UCA} | 41 | mG*mA*mU*GGGCACCACCCCGGUGAACAGCUCCUCG CCCUUGCUCACUGGCAGAGCCCUUCAGCAUCGCGAGC AGGCGCUGCCmU*mC*mC |
| arRNA_{ACA} | 42 | mG*mA*mU*GGGCACCACCCCGGUGAACAGCUCCUCG CCCUUGCUCACUGGCAGAGCCCUACAGCAUCGCGAGC AGGCGCUGCCmU*mC*mC |
| arRNA_{GCA} | 43 | mG*mA*mU*GGGCACCACCCCGGUGAACAGCUCCUCG CCCUUGCUCACUGGCAGAGCCCUGCAGCAUCGCGAGC AGGCGCUGCCmU*mC*mC |
| arRNA_{CCA} | 44 | mG*mA*mU*GGGCACCACCCCGGUGAACAGCUCCUCG CCCUUGCUCACUGGCAGAGCCCUCCAGCAUCGCGAGC AGGCGCUGCCmU*mC*mC |
| arRNA_{UCG} | 45 | mG*mA*mU*GGGCACCACCCCGGUGAACAGCUCCUCG CCCUUGCUCACUGGCAGAGCCCUUCGGCAUCGCGAGC |
| | | AGGCGCUGCCmU*mC*mC |
| arRNA_{ACG} | 46 | mG*mA*mU*GGGCACCACCCCGGUGAACAGCUCCUCG CCCUUGCUCACUGGCAGAGCCCUACGGCAUCGCGAGC AGGCGCUGCCmU*mC*mC |
| arRNA_{GCG} | 47 | mG*mA*mU*GGGCACCACCCCGGUGAACAGCUCCUCG CCCUUGCUCACUGGCAGAGCCCUGCGGCAUCGCGAGC AGGCGCUGCCmU*mC*mC |
| arRNA_{CCG} | 48 | mG*mA*mU*GGGCACCACCCCGGUGAACAGCUCCUCG CCCUUGCUCACUGGCAGAGCCCUCCGGCAUCGCGAGC AGGCGCUGCCmU*mC*mC |
| arRNA_{UCC} | 49 | mG*mA*mU*GGGCACCACCCCGGUGAACAGCUCCUCG CCCUUGCUCACUGGCAGAGCCCUUCCGCAUCGCGAGC AGGCGCUGCCmU*mC*mC |
| arRNA_{ACC} | 50 | mG*mA*mU*GGGCACCACCCCGGUGAACAGCUCCUCG CCCUUGCUCACUGGCAGAGCCCUACCGCAUCGCGAGC AGGCGCUGCCmU*mC*mC |
| arRNA_{GCC} | 51 | mG*mA*mU*GGGCACCACCCCGGUGAACAGCUCCUCG CCCUUGCUCACUGGCAGAGCCCUGCCGCAUCGCGAGC AGGCGCUGCCmU*mC*mC |
| arRNAccc | 52 | mG*mA*mU*GGGCACCACCCCGGUGAACAGCUCCUCG CCCUUGCUCACUGGCAGAGCCCUCCCGCAUCGCGAGC AGGCGCUGCCmU*mC*mC |
| arRNA_{random} | 53 | mC*mA*mA*UAGGCACUAACUUAUUGGCGCUGGUGAA CGGACUUCCUCUCGAGUACCAGAAGAUGACUACAAA ACUCCUUUCCAUUGCGAGUAUmC*mG*mG |

| | | |
|---|---|---|
| Note: m indicates that the right base is subjected to a dimethyl oxygen modification (2'-O-Me); * indicates that the adjacent two nucleotides are linked through a phosphorothioate bond; and the underlined nucleic acids are 3 bases directly opposite to the three-base motif in the target RNA when the arRNA hybridizes to the target RNA. | | |

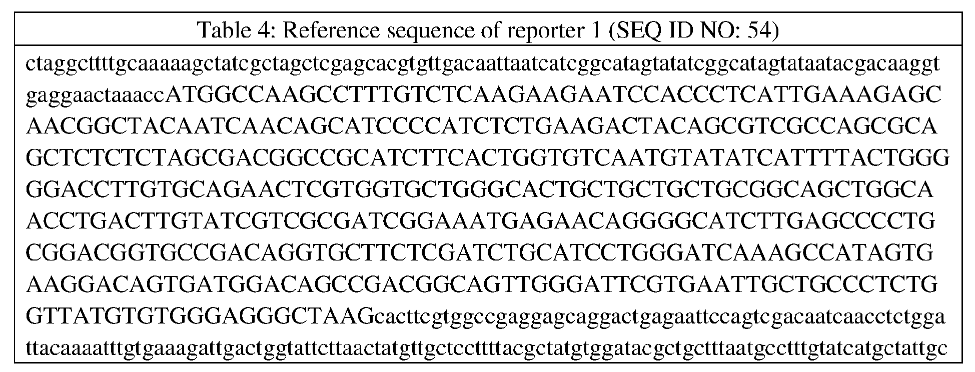

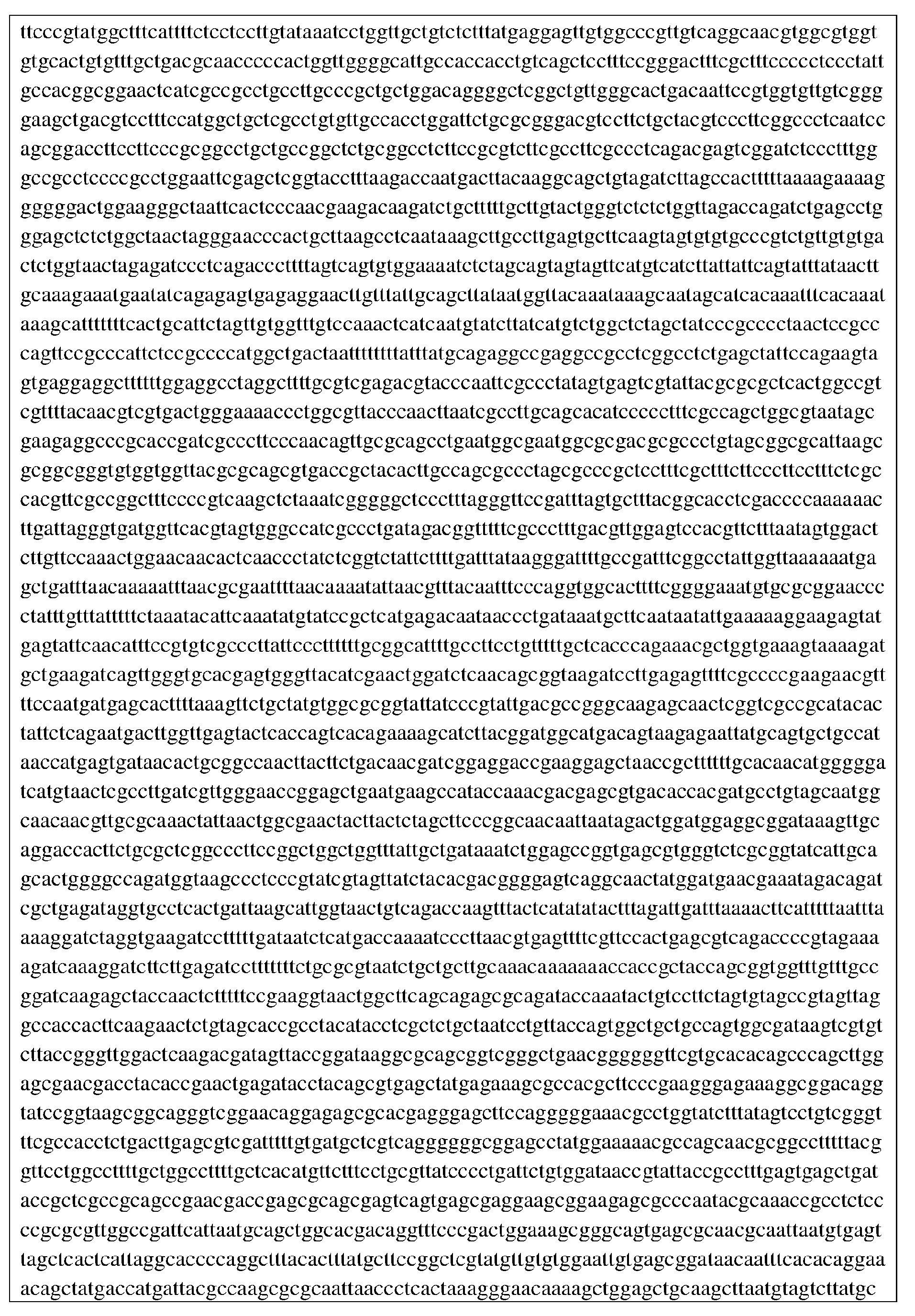

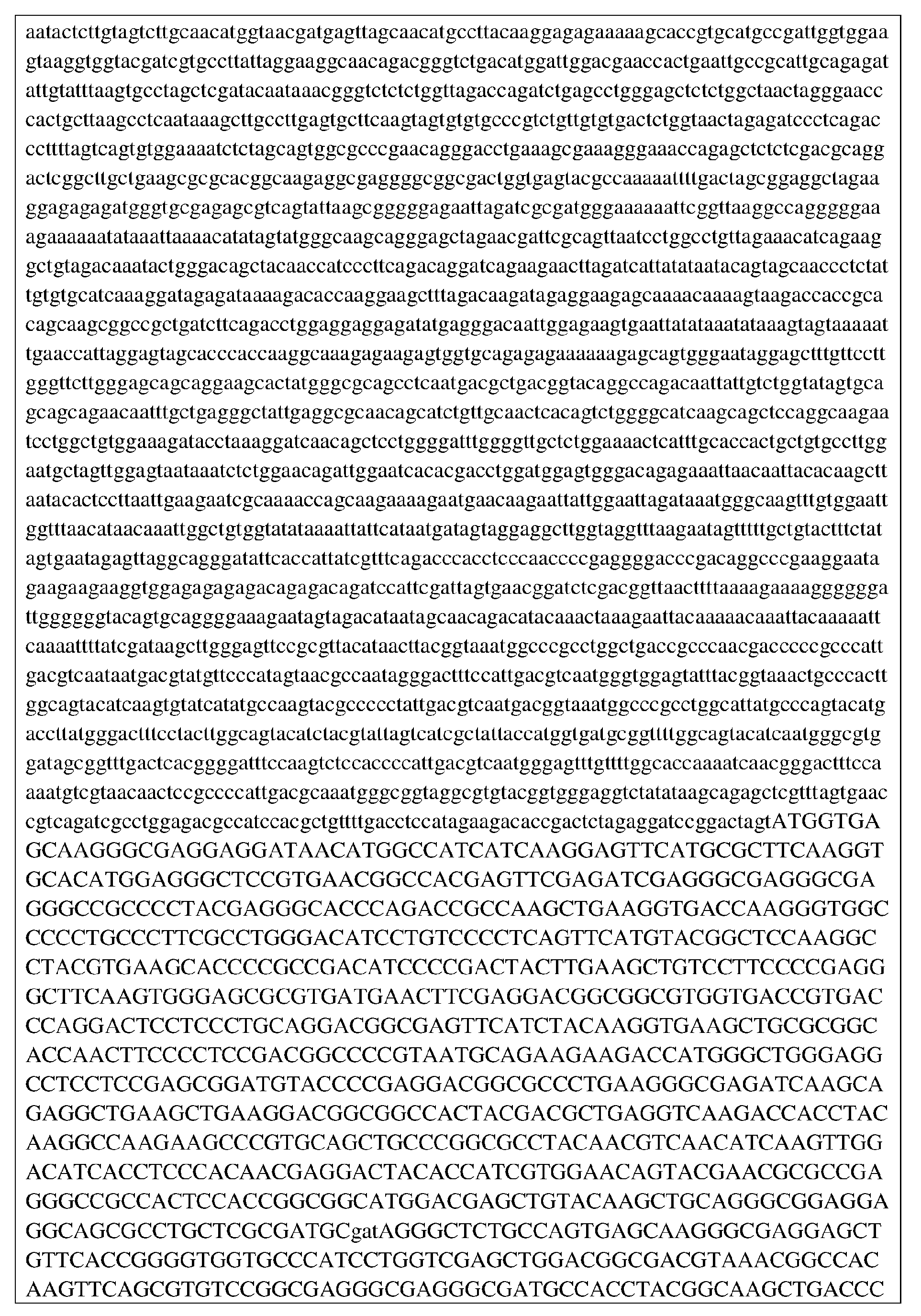

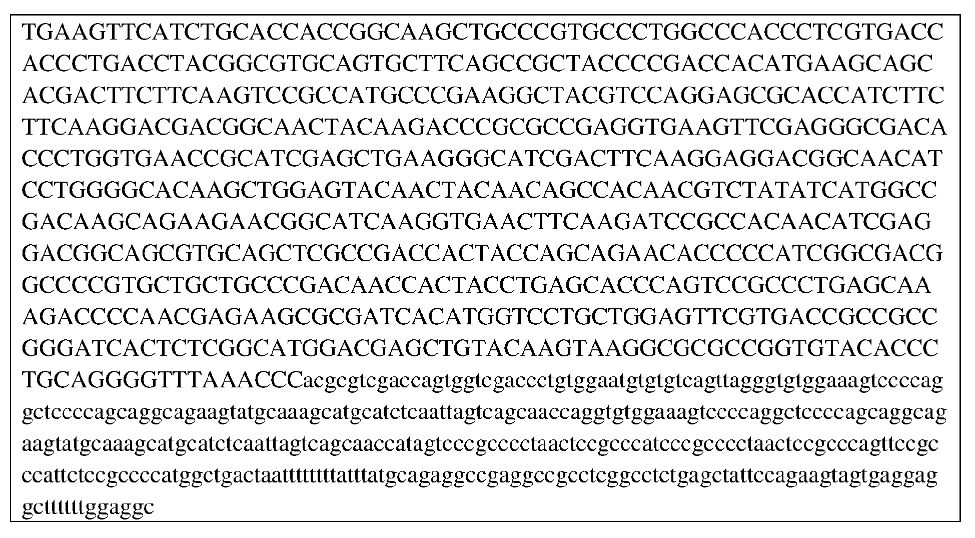

**Table 5:**

| RNA | Corresponding arRNA | RNA sequence |
|---|---|---|
| A^{∗} | arRNA_{UAG} | gcugcuucauguggucgggguagcggcugaagcacugcacgccguAggucaggg uggucacgagggugggccagggcacgggcagcuugcc (SEQ ID NO: 55) gcugcuucauguggucgggguagcggcugaagcacugcacgccguUggucaggg uggucacgagggugggccagggcacgggcagcuugcc (SEQ ID NO: 56) gcugcuucauguggucgggguagcggcugaagcacugcacgccguCggucaggg uggucacgagggugggccagggcacgggcagcuugcc (SEQ ID NO: 57) gcugcuucauguggucgggguagcggcugaagcacugcacgccguGggucaggg uggucacgagggugggccagggcacgggcagcuugcc (SEQ ID NO: 58) |
| U^{∗} | arRNA_{UUG} | |
| C^{∗} | arRNA_{UCG} | |
| G^{∗} | arRNA_{UGG} | |

| | | |
|---|---|---|
| Note: there is no difference between capital and small letters, and capital letters are only used for highlighting differences between sequences. | | |

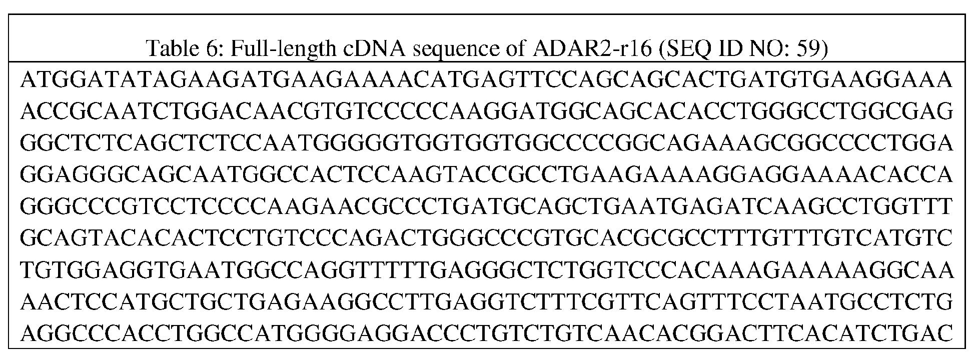

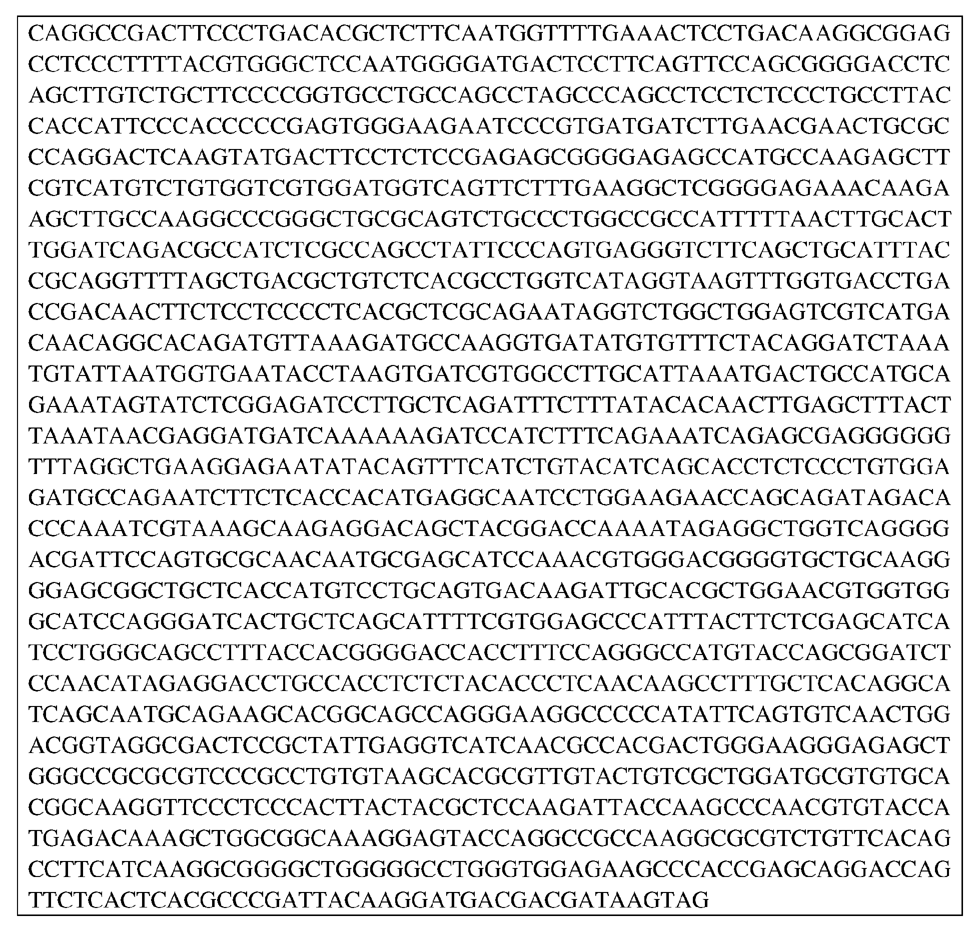

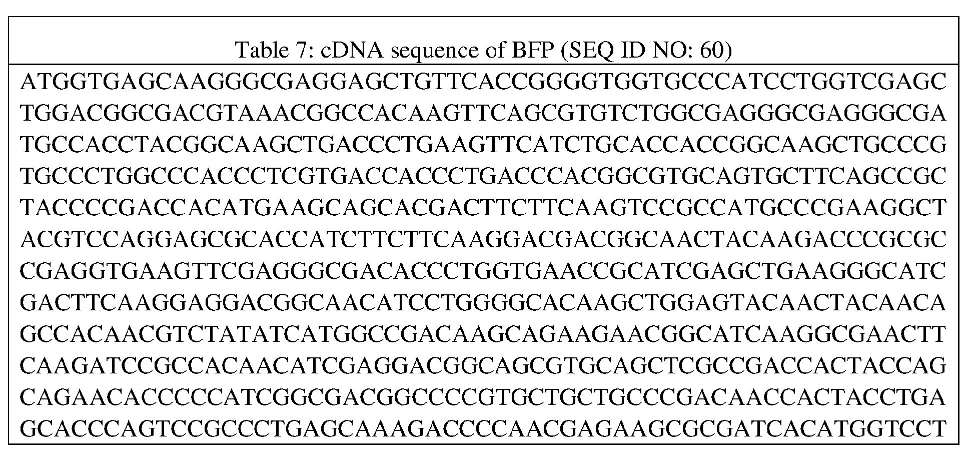

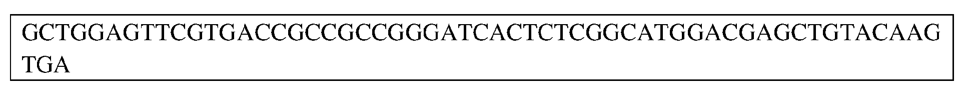

**Table 8: Relevant arRNA sequences used in Example 4**

| Corresponding arRNA | SEQ ID NO: | RNA sequence |
|---|---|---|
| arRNA_{UUU} | 61 | gcugcuucaugugggucggguagcggcugaagcacugcacgccgUUUgucagggug gucacgaggugggccagggcacgggcagcuugcc |
| arRNA_{AUU} | 62 | gcugcuucaugugggucggguagcggcugaagcacugcacgccgAUUgucagggug gucacgaggguggggccagggcacgggcagcuugcc |
| arRNA_{GUU} | 63 | gcugcuucauguggucgggguagcggcugaagcacugcacgccgGUUgucagggug gucacgagggugggccagggcacgggcagcuugcc |
| arRNA_{CUU} | 64 | gcugcuucaugugggucggguagcggcugaagcacugcacgccgCUUgucagggug gucacgaggugggccagggcacgggcagcuugcc |
| arRNA_{UUA} | 65 | gcugcuucaugugggucggguagcggcugaagcacugcacgccgUUAgucagggug gucacgaggugggccagggcacgggcagcuugcc |
| arRNA_{AUA} | 66 | gcugcuucaugugggucggguagcggcugaagcacugcacgccgAUAgucagggug gucacgaggugggccagggcacgggcagcuugcc |
| arRNA_{GUA} | 67 | gcugcuucaugugggucggguagcggcugaagcacugcacgccgGUAgucagggug gucacgaggugggccagggcacgggcagcuugcc |
| arRNA_{CUA} | 68 | gcugcuucaugugggucggguagcggcugaagcacugcacgccgCUAgucagggug gucacgaggugggccagggcacgggcagcuugcc |
| arRNA_{UUG} | 56 | gcugcuucauguggucgggguagcggcugaagcacugcacgccgUUGgucagggug gucacgagggugggccagggcacgggcagcuugcc |
| arRNA_{AUG} | 69 | gcugcuucaugugggucggguagcggcugaagcacugcacgccgAUGgucagggug gucacgaggguggccagggcacgggcagcuugcc |
| arRNA_{GUG} | 70 | gcugcuucaugugguucggguagcggcugaagcacugcacgccgGUGgucagggug gucacgaggugggccagggcacgggcagcuugcc |
| arRNA_{CUG} | 71 | gcugcuucauguggucgggguagcggcugaagcacugcacgccgCUGgucagggug gucacgagggugggccagggcacgggcagcuugcc |
| arRNA_{UUC} | 72 | gcugcuucaugugggucggguagcggcugaagcacugcacgccgUUCgucagggug gucacgaggugggccagggcacgggcagcuugcc |
| arRNA_{AUC} | 73 | gcugcuucaugugggucggguagcggcugaagcacugcacgccgAUCgucagggug gucacgaggugggccagggcacgggcagcuugcc |
| arRNA_{GUC} | 74 | gcugcuucaugugggucggguagcggcugaagcacugcacgccgGUCgucagggug gucacgaggugggccagggcacgggcagcuugcc |
| arRNA_{CUC} | 75 | gcugcuucaugguggucggguagcggcugaagcacugcacgccgCUCgucagggug gucacgaggugggccagggcacgggcagcuugcc |
| Ran-91 | 76 | uaauccugaauaucgcgcaauuccccagcagagaacaucgcggugugaacgucccuuua uaccgggcagguauagcugaaaucagcguggc |

| | | |
|---|---|---|
| Note: there is no difference between capital and small letters, and capital letters are only used for highlighting three consecutive complementary bases. | | |

### References:

*1.* Adikusuma, F., Piltz, S., Corbett, M. A., Turvey, M., McColl, S. R., Helbig, K. J., ... & Thomas, P. Q. (2018). Large deletions induced by Cas9 cleavage. Nature, 560(7717), E8-E9*.*
*2.* Cox, D. B., Gootenberg, J. S., Abudayyeh, O. O., Franklin, B., Kellner, M. J., Joung, J., & Zhang, F. (2017). RNA editing with CRISPR-Cas13. Science, 358(6366), 1019-1027*.*
*3.* Charlesworth, C. T., Deshpande, P. S., Dever, D. P., Camarena, J., Lemgart, V. T., Cromer, M. K., ... & Behlke, M. A. (2019). Identification of preexisting adaptive immunity to Cas9 proteins in humans. Nature medicine, 25(2), 249-254*.*
*4.* Cullot, G., Boutin, J., Toutain, J., Prat, F., Pennamen, P., Rooryck, C., ... & Bibeyran, A. (2019). CRISPR-Cas9 genome editing induces megabase-scale chromosomal truncations. Nature communications, 10(1), 1-14*.*
*5.* Enache, O. M., Rendo, V., Abdusamad, M., Lam, D., Davison, D., Pal, S., ... & Thorner, A. R. (2020). Cas9 activates the p53 pathway and selects for p53-inactivating mutations. Nature Genetics, 1-7*.*
*6.* Haapaniemi, E., Botla, S., Persson, J., Schmierer, B., & Taipale, J. (2018). CRISPR-Cas9 genome editing induces a p53-mediated DNA damage response. Nature medicine, 24(7), 927-930*.*
*7.* Merkle, T., Merz, S., Reautschnig, P., Blaha, A., Li, Q., Vogel, P., ... & Stafforst, T. (2019). Precise RNA editing by recruiting endogenous ADARs with antisense oligonucleotides. Nature biotechnology, 37(2), 133-138*.*
*8.* Qu, L., Yi, Z., Zhu, S., Wang, C., Cao, Z., Zhou, Z., ... & Bao, Y. (2019). Programmable RNA editing by recruiting endogenous ADAR using engineered RNAs. Nature biotechnology, 37(9), 1059-1069*.*
*9.* Vogel, P., Moschref, M., Li, Q., Merkle, T., Selvasaravanan, K. D., Li, J. B., & Stafforst, T. (2018). Efficient and precise editing of endogenous transcripts with SNAP-tagged ADARs. Nature methods, 15(7), 535-538*.*
*10.* Nishikura K. (2010). Functions and regulation of RNA editing by ADAR deaminases. Annual review of biochemistry, 79, 321-349*.*
*11.* Paul Vogel, Matin Moschref, Qin Li, Tobias Merkle, Karthika D. Selvasaravanan, Jin Billy Li & Thorsten Stafforst. (2018). Efficient and precise editing of endogenous transcripts with SNAP-tagged ADARs. Nat Methods 15, 535-538 (2018*).*
*12.* Abudayyeh, O. O., Gootenberg, J. S., Franklin, B., Koob, J., Kellner, M. J., Ladha, A., ... & Zhang, F. (2019). A cytosine deaminase for programmable single-base RNA editing. Science, 365(6451), 382-386*.*
*13.* Vu, L. T., Nguyen, T. T. K., Md Thoufic, A. A., Suzuki, H., & Tsukahara, T. (2016). Chemical RNA editing for genetic restoration: the relationship between the structure and deamination efficiency of carboxyvinyldeoxyuridine oligodeoxynucleotides. Chemical biology & drug design, 87(4), 583-593*.*
*14.* Keppler, A., Gendreizig, S., Gronemeyer, T., Pick, H., Vogel, H., & Johnsson, K. (2003). A general method for the covalent labeling of fusion proteins with small molecules in vivo. Nature Biotechnology, 21(1), 86-89*.*
*15.* Stafforst, T., & Schneider, M. F. (2012). An RNA-Deaminase Conjugate Selectively Repairs Point Mutations. Angewandte Chemie, 51(44), 11166-11169*.*

## Claims

1. A method for editing target RNA at a target residue position in a host cell, which comprises introducing ADAR-recruiting RNA (arRNA) or a construct encoding the arRNA into a host cell, wherein the arRNA comprises a complementary RNA sequence that hybridizes to a target RNA; the target residue is located in a three-base motif comprising a 5' nearest-neighbor residue (upstream residue) of the target residue in the target RNA, the target residue, and a 3' nearest-neighbor residue (downstream residue) of the target residue in the target RNA, wherein the three-base motif is not UAG, and the complementary RNA sequence comprises a mismatch directly opposite to the upstream residue and/or the downstream residue in the target RNA.

2. The method according to claim 1, wherein the complementary RNA sequence comprises a mismatch directly opposite to the upstream residue in the target RNA.

3. The method according to claim 1 or 2, wherein the complementary RNA sequence comprises a mismatch directly opposite to the downstream residue in the target RNA.

4. The method according to any one of claims 1 to 3, wherein the target residue is adenosine.

5. The method according to claim 4, wherein the upstream residue is selected from the group consisting of: G, A, and C.

6. The method according to any one of claims 1 to 4, wherein the three-base motif is selected from the group consisting of: GAG, GAC, GAA, GAU, AAG, AAC, AAA, AAU, CAG, CAC, CAA, CAU, UAA, UAC, and UAU.

7. The method according to any one of claims 4 to 6, wherein the complementary RNA sequence comprises cytidine, adenosine or uridine directly opposite to the target adenosine in the target RNA.

8. The method according to any one of claims 4 to 7, wherein the complementary RNA sequence further comprises one or more mismatches respectively opposite to a non-target adenosine in the target RNA.

9. The method according to any one of claims 4 to 8, wherein the three-base motif is GAU, and the complementary RNA sequence comprises ACG, UCC, CCU or ACA opposite to the three-base motif.

10. The method according to claim 9, wherein the three-base motif is GAU, and the complementary RNA sequence comprises ACG opposite to the three-base motif.

11. The method according to any one of claims 4 to 8, wherein the three-base motif is GAA, and the complementary RNA sequence comprises UCA, CCG, CCC or UCG opposite to the three-base motif.

12. The method according to claim 11, wherein the three-base motif is GAA, and the complementary RNA sequence comprises UCA or UCG opposite to the three-base motif.

13. The method according to any one of claims 4 to 8, wherein the three-base motif is GAC, and the complementary RNA sequence comprises GCG or GCA opposite to the three-base motif.

14. The method according to claim 13, wherein the three-base motif is GAC, and the complementary RNA sequence comprises GCG opposite to the three-base motif.

15. The method according to any one of claims 4 to 8, wherein the three-base motif is GAG, and the complementary RNA sequence comprises CCG, CCA, CCC, UCC or UCG opposite to the three-base motif.

16. The method according to claim 15, wherein the three-base motif is GAG, and the complementary RNA sequence comprises CCG opposite to the three-base motif.

17. The method according to any one of claims 4 to 8, wherein the upstream residue in the target RNA is a nucleotide selected from the group consisting of: G, C, A, and U, and the preferred order is as follows: G>C≈A>U.

18. The method according to any one of claims 4 to 8, wherein the upstream residue in the three-base motif is G, and the base opposite to the upstream residue in the complementary RNA is G or A.

19. The method according to any one of claims 4 to 18, wherein the downstream residue in the three-base motif is strictly complementary to an opposite base in the complementary RNA.

20. The method according to any one of claims 4 to 19, wherein the arRNA recruits an adenosine deaminase acting on RNA (ADAR) or a fusion protein comprising an ADAR catalytic domain to deaminate the target adenosine in the target RNA.

21. The method according to claim 20, wherein the fusion protein comprising an ADAR catalytic domain further comprises a targeting domain.

22. The method according to claim 20 or 21, wherein the ADAR protein or the fusion protein comprising an ADAR catalytic domain, or a construct encoding the ADAR protein or the fusion protein comprising an ADAR catalytic domain is exogenously introduced into the host cell.

23. The method according to claim 20, wherein the ADAR protein is endogenously expressed by the host cell.

24. The method according to any one of claims 1 to 3, wherein the target residue is cytidine, and the arRNA recruits a deaminase acting on RNA and having cytidine deaminase activity to deaminate the target cytidine in the target RNA.

25. The method according to claim 24, wherein the three-base motif in which the target cytidine in the target RNA is located is any one selected from the group consisting of: GCG, GCC, GCA, GCU, ACG, ACC, ACA, ACU, CCG, CCC, CCA, CCU, UCA, UCC, UCU, and UCG.

26. The method according to claim 24 or 25, wherein the complementary RNA sequence comprises cytidine, adenosine or uridine opposite to the target cytidine in the target RNA.

27. The method according to any one of claims 24 to 26, wherein the complementary RNA sequence further comprises one or more mismatches respectively opposite to a non-target cytidine in the target RNA.

28. The method according to any one of claims 24 to 27, wherein the upstream residue in the three-base motif is G, and the base opposite to the upstream residue in the complementary RNA is G.

29. The method according to any one of claims 24 to 27, wherein the three-base motif is GCA, and the complementary RNA sequence comprises UUG or UCG opposite to the three-base motif.

30. The method according to claim 29, wherein the three-base motif is GCA, and the complementary RNA sequence comprises UUG opposite to the three-base motif.

31. The method according to any one of claims 24 to 27, wherein the three-base motif is CCA, and the complementary RNA sequence comprises AUG opposite to the three-base motif.

32. The method according to any one of claims 24 to 27, wherein the upstream residue in the target RNA is a nucleotide selected from the group consisting of: G, C, A, and U, and the preferred order is as follows: G>C>A≈U.

33. The method according to any one of claims 24 to 32, wherein the deaminase with the cytidine deaminase activity is a deaminase with the C-to-U catalytic activity obtained by gene modification of ADAR protein or a fusion protein comprising an ADAR catalytic domain.

34. The method according to claim 33, wherein the deaminase with the cytidine deaminase activity further comprises a targeting domain.

35. The method according to claim 21 or 34, wherein the targeting domain is any one selected from the group consisting of: SNAP-tag, λN peptide, and Cas13 protein without catalytic activity.

36. The method according to claim 22 or 24, wherein the construct is any one selected from the group consisting of: a virus vector, a plasmid, and a linear nucleic acid chain.

37. The method according to any one of claims 1 to 36, wherein the arRNA has a length of about 20-260 nucleotides.

38. The method according to any one of claims 1 to 37, wherein the arRNA is single-stranded RNA.

39. The method according to any one of claims 1 to 38, wherein the complementary RNA sequence is single-stranded, and the arRNA further comprises one or more double-stranded regions.

40. The method according to any one of claims 1 to 39, wherein the arRNA further comprises an ADAR-recruiting domain.

41. The method according to any one of claims 1 to 40, wherein the arRNA comprises one or more chemical modifications.

42. The method according to any one of claims 1 to 40, wherein the arRNA does not comprise any chemical modification.

43. The method according to any one of claims 1 to 42, wherein the target RNA is RNA selected from the group consisting of: messenger RNA precursor, messenger RNA, ribosome RNA, transfer RNA, long non-coding RNA, and small RNA.

44. The method according to any one of claims 1 to 43, wherein the target residue in the target RNA is edited to result in a missense mutation, premature termination codon, aberrant splicing or alternative splicing in the target RNA, alternatively, to reverse a missense mutation, premature termination codon, aberrant splicing or alternative splicing in the target RNA.

45. The method according to any one of claims 1 to 44, wherein the target residue in the target RNA is edited to result in a point mutation, truncation, extension and/or misfolding of a protein encoded by the target RNA, alternatively, to obtain a functional, full-length, correctly folded and/or wild-type protein by reversing a missense mutation, premature termination codon, aberrant splicing or alternative splicing in the target RNA.

46. The method according to any one of claims 1 to 45, wherein the host cell is a eukaryocyte.

47. The method according to claim 46, wherein the host cell is a mammalian cell.

48. The method according to claim 47, wherein the host cell is a human or mouse cell.

49. An edited RNA produced by the method according to any one of claims 1 to 48, or a host cell comprising the edited RNA.

50. A library, which comprises a plurality of RNAs according to claim 49, or a plurality of host cells comprising the edited RNA according to claim 49.

51. A method for treating or preventing a disease or condition in an individual, comprising editing target RNA associated with the disease or condition in cells of the individual by the method according to any one of claims 1 to 48.

52. The method according to claim 51, wherein the disease or condition is an inherited gene disease or a disease or condition associated with one or more acquired gene mutations.

53. An arRNA, comprising the arRNA used in the method according to any one of claims 1 to 49.

54. A virus vector, plasmid or linear nucleic acid chain, comprising the arRNA according to claim 53, wherein the arRNA does not comprise any chemical modification.

55. A library, comprising a plurality of arRNAs according to claim 53, or a plurality of virus vectors, plasmids or linear nucleic acids according to claim 54.

56. A composition, comprising the arRNA according to claim 53, or the virus vector, plasmid or linear nucleic acid chain according to claim 54.

57. A host cell, comprising the arRNA according to claim 53, or the virus vector, plasmid or linear nucleic acid chain according to claim 54.
